# EUROPEAN PATENT SPECIFICATION

(11) **EP 3 164 195 B1**
(45) Date of publication and mention of the grant of the patent: **15.06.2022**
(21) Application number: 15814655.5
(22) Date of filing: 03.07.2015
(51) Int. Cl.: A61K 31/501, A61K 31/433, A61K 31/4439, A61P 35/00

(54) **GLUTAMINASE INHIBITOR THERAPY**
GLUTAMINASE-HEMMERTHERAPIE
THÉRAPIE PAR INHIBITEUR DE GLUTAMINASE

(30) Priority: 03.07.2014 US 201462020731 P; 03.07.2014 US 201462020539 P; 03.07.2014 US 201462020519 P; 03.07.2014 US 201462020524 P; 30.06.2015 US 201562187160 P; 02.07.2015 US 201514791186; 02.07.2015 US 201514791206
(43) Date of publication of application: 10.05.2017
(73) Proprietor: Board of Regents, The University of Texas System, Austin, TX 78701 (US)
(72) Inventor: HEFFERNAN, Timothy, Sugarl Land, TX 77479 (US); TONIATTI, Carlo, Houston, TX 77005 (US); KOVACS, Jeffrey, Pearland, TX 77584 (US); GIULIANI, Virginia, Missouri City TX 77459 (US); SPENCER, Nakia, Houston, TX 77021 (US); DI FRANCESCO, Maria Emilia, Houston, TX 77008 (US); BRISTOW, Christopher A., Houston, TX 77054 (US)
(74) Representative: Bridle, Andrew Barry
(86) International application number: PCT/US2015/039153
(87) International publication number: WO 2016/004418

(56) References cited:
- WO-A1-2014/078645
- WO-A2-2011/143160
- US-A1- 2010 255 117
- US-A1- 2010 255 117
- BARAK ROTBLAT ET AL: "NRF2 and p53: Januses in cancer?", ONCOTARGET, vol. 3, no. 11, 1 January 2012 (2012-01-01), pages 1272-1283, XP055251330, DOI: 10.18632/oncotarget.754
- M. I. GROSS ET AL: "Antitumor Activity of the Glutaminase Inhibitor CB-839 in Triple-Negative Breast Cancer", MOLECULAR CANCER THERAPEUTICS, vol. 13, no. 4, 12 February 2014 (2014-02-12), pages 890-901, XP055243589, US ISSN: 1535-7163, DOI: 10.1158/1535-7163.MCT-13-0870
- K. THANGAVELU ET AL: "Structural basis for the allosteric inhibitory mechanism of human kidney-type glutaminase (KGA) and its regulation by Raf-Mek-Erk signaling in cancer cell metabolism", PROCEEDINGS NATIONAL ACADEMY OF SCIENCES PNAS, vol. 109, no. 20, 26 April 2012 (2012-04-26), pages 7705-7710, XP055098262, US ISSN: 0027-8424, DOI: 10.1073/pnas.1116573109
- SINGH A ET AL: "Dysfunctional KEAP1-NRF2 interaction in non-small-cell lung cancer", PLOS MEDICINE, PUBLIC LIBRARY OF SCIENCE, US, vol. 3, no. 10, E420, 1 October 2006 (2006-10-01), pages 1865-1876, XP003015746, ISSN: 1549-1676, DOI: 10.1371/JOURNAL.PMED.0030420
- P. ZHANG ET AL: "Loss of Kelch-Like ECH-Associated Protein 1 Function in Prostate Cancer Cells Causes Chemoresistance and Radioresistance and Promotes Tumor Growth", MOLECULAR CANCER THERAPEUTICS, vol. 9, no. 2, 1 February 2010 (2010-02-01), pages 336-346, XP055227547, US ISSN: 1535-7163, DOI: 10.1158/1535-7163.MCT-09-0589
- ROTBLAT ET AL.: 'NRF2 and p53: Januses in cancer?' ONCOTARGET vol. 3, no. 11, 19 November 2012, pages 1272 - 1283, XP055251330
- ZHANG ET AL.: 'Loss of Kelch-Like ECH-Associated Protein 1 Function in Prostate Cancer Cells Causes Chemoresistance and Radioresistance and Promotes Tumor Growth' MOLECULAR CANCER THERAPEUTICS vol. 9, no. 2, 01 February 2010, pages 336 - 347, XP055227547
- INAMI ET AL.: 'Persistent activation of Nrf2 through p62 in hepatocellular carcinoma cells' THE JOURNAL OF CELL BIOLOGY vol. 193, no. 2, 01 April 2011, pages 275 - 284, XP055158643
- ZHANG ET AL.: 'Distinct Cysteine Residues in Keap1 Are Required for Keap1-Dependent Ubiquitination of Nrf2 and for Stabilization of Nrf2 by Chemopreventive Agents and Oxidative Stress' MOLECULAR AND CELLULAR BIOLOGY vol. 23, no. 22, 01 November 2003, pages 8137 - 8151, XP055251345
- THANGAVELU ET AL.: 'Structural basis for the allosteric inhibitory mechanism of human kidney-type glutaminase (KGA) and its regulation by Raf-Mek-Erk signaling in cancer cell metabolism' PROC. NATL. ACAD. SCI. USA vol. 109, no. 20, 15 May 2012, pages 7705 - 7710, XP055251346
- LI ET AL.: 'Sulforaphane Potentiates the Efficacy of 17-Allylamino 17- Demethoxygeldanamycin Against Pancreatic Cancer Through Enhanced Abrogation of Hsp90 Chaperone Function' NUTRITION AND CANCER vol. 63, no. 7, 29 August 2011, pages 1151 - 1159, XP055167208
- ROBINSON ET AL.: 'Novel mechanism of inhibition of rat kidney-type glutaminase by bis-2-(5-phenylacetamido-1,2,4-thiadiazol-2 -yl)ethyl sulfide (BPTES).' THE BIOCHEMICAL JOURNAL vol. 406, 15 September 2007, pages 407 - 414, XP055069615

## Description

This application claims the benefit of priority of United States Application 14/791,284 filed July 3, 2015, which claims the benefit of United States Provisional Application No. 62/020,519, filed July 3, 2014; and of United States Application No. 14/791,206, filed July 2, 2015, which claims the benefit of United States Provisional Application No 62/020,524, filed July 3, 2014; and of United States Application No. 14/791,186, filed July 2, 2015, which claims the benefit of United States Provisional Application No. 62/020,539, filed July 3, 2014; and of United States Provisional Application Nos. 62/020,731, filed July 3, 2014; and of 62/187,160 filed June 30, 2015.

Metabolic deregulation is a hallmark of cancer as tumors exhibit an increased demand for nutrients and macromolecules to fuel their rapid proliferation. Glutamine (Gln), the most abundant amino acid in circulation, plays an essential role in providing cancer cells with biosynthetic intermediates required to support proliferation and survival. Specifically, glutaminolysis, or the enzymatic conversion of glutamine to glutamate, provides proliferating cancer cells with a source of nitrogen for amino acid and nucleotide synthesis, and a carbon skeleton to fuel ATP and NADPH synthesis through the TCA cycle.

In addition to supporting cell growth, glutamine metabolism plays a critical role in maintaining cellular redox homeostasis as glutamate can be converted into glutathione, the major intracellular antioxidant. Cancer cells require a constant source of biomass and macro-molecules to support cell division and reducing agents to maintain redox homeostasis. While many of these building blocks are provided through aerobic glycolysis, many cancer cells have evolved a dependence on glutamine metabolism for growth and survival.

Glutamine metabolism, *i.e.,* glutaminolysis is regulated by mitochondrial glutaminase (GLS), the rate limiting enzyme that catalyzes the conversion of glutamine to glutamate and ammonia. Mammalian cells contain two genes that encode glutaminase: the kidney-type (GLS-1) and liver-type (GLS-2) enzymes. Each has been detected in multiple tissue types, with GLS-1 being widely distributed throughout the body. GLS-1 is a phosphate-activated enzyme that exists in humans as two major splice variants, a long form (referred to as KGA) and a short form (GAC), which differ only in their C-terminal sequences. Both forms of GLS-1 are thought to bind to the inner membrane of the mitochondrion in mammalian cells, although at least one report suggests that glutaminase may exist in the intramembrane space, dissociated from the membrane. GLS is frequently overexpressed in human tumors and has been shown to be positively regulated by oncogenes such as Myc. Consistent with the observed dependence of cancer cell lines on glutamine metabolism, pharmacological inhibition of GLS offers the potential to target Gln addicted tumors. Such targeted treatment, however, is hampered by the lack of clinical biomarkers to identify sensitive patient populations.

Singh et al 'Dysfunctional KEAP1-NRF2 interactions in non-small cell lung cancer: PLOS Medicine, vol 3, no 10, October 2006, pages 1865-1876 describes that dysfunctional KEAP1-NRF2 interaction is frequently seen in non small cell lung cancer. The loss of KEAP1 function leads to activation of NRF2 mediated gene expression in cancer cells.

Thus, there is a need for a means to identify markers and mechanisms that provide a method for evaluating a patient's successful treatment with glutaminase inhibitors as well as for methods of such treatment.

The invention is based, in part, on the discovery of biomarkers and mechanisms that indicate a cancer patient's positive response to treatment with glutaminase inhibitors and methods to treat such patients. Accordingly, in one aspect which is not part of the present invention, the disclosure comprises evaluating whether to administer a compound that inhibits glutathione production to a subject by determining the presence of certain biomarkers or mechanisms and administering such a compound to the patient. In another aspect which is not part of the present invention, the disclosure also comprises methods of treating patients identified by the presence of the biomarkers or mechanisms. Throughout, the term 'method of treatment' should be read as compounds/compositions/medicaments for use in the treatment of lung cancer in the human body.

In one aspect which is not part of the present invention, the disclosure comprises a method of treatment of a subject having a disorder in need of treatment comprising administering a compound that inhibits glutathione production to a subject. The method comprises optionally obtaining a biological sample from a subject, determining that the NRF2/KEAP1 pathway in said subject is deregulated, or that NRF2 signaling in said subject is hyperactive, or determining the presence of a loss-of-function mutation in KEAP1 or a gain-of-function mutation in NRF2 of said subject, or an increase in the intracellular concentration of glutathione in said subject and determining that the compound that inhibits glutathione production should be administered to the subject.

In another aspect which is not part of the present invention, the disclosure comprises a method of treating a subject in need of treatment. The method comprises determining that the NRF2/KEAP1 pathway in said subject is deregulated, that NRF2 signaling in said subject is hyperactive, that the nucleic acid of said subject comprises a loss-of-function mutation in KEAP1 or a gain-of-function mutation in NRF2, or determining an increase in the intracellular concentration of glutathione in said subjectand administering a glutaminase inhibitor to the subject. The subject in need of treatment may be afflicted with lung cancer. The glutaminase inhibitor may, for example, be a GLS-1 inhibitor or a selective inhibitor of GLS-1.

According to an embodiment of the invention, the invention comprises a glutaminase inhibitor according to the claims for use in the treatment of lung cancer, wherein the subject has: a deregulated NRF2/KEAP1 pathway;hyperactive NRF2 signaling; a loss-of-function mutation in the KEAP1 nucleic acid; a a gain-of-function mutation in NRF2 nucleic acid; or an increase in intracellular concentration of glutathione.

### BRIEF DESCRIPTION OF FIGURES

The Figures disclose studies with a compound IACS-011393; said compound is a reference example and it falls outside the scope of the invention.

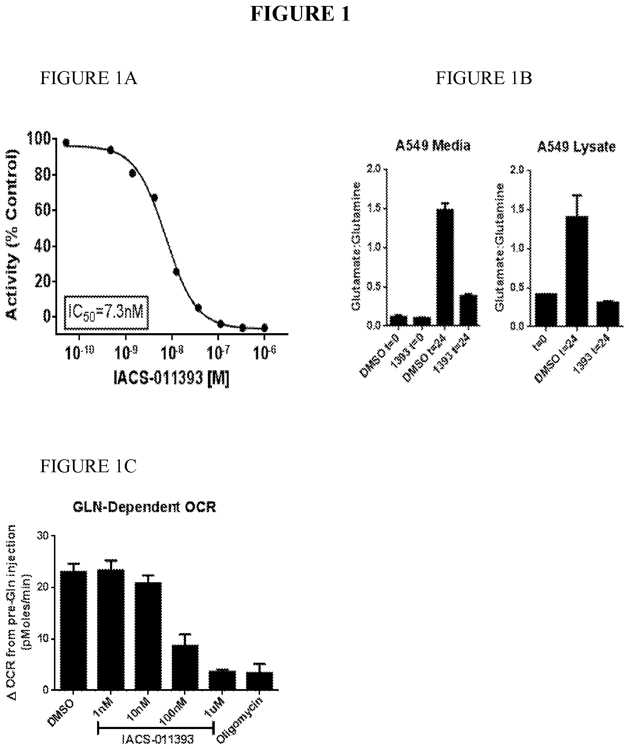

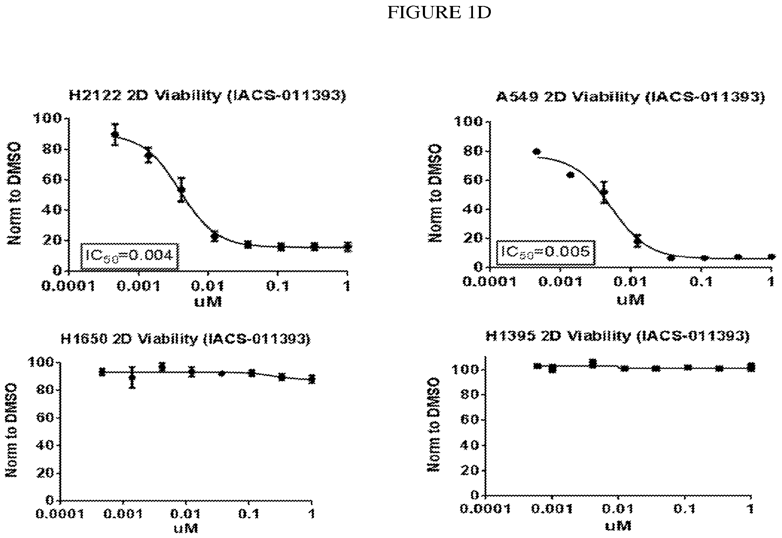

Figure 1 shows the inhibition of GLS by IACS-011393. Figure 1A shows the inhibition of GAC by varying concentrations of IACS-011393. Figure 1B shows the target engagement measured after treatment with IACS-011393. Figure 1C shows that A549 cells utilize glutamine anaplerosis to drive respiration. Figure 1D shows the different sensitivities of different NSCLC lines to IACS-011393.

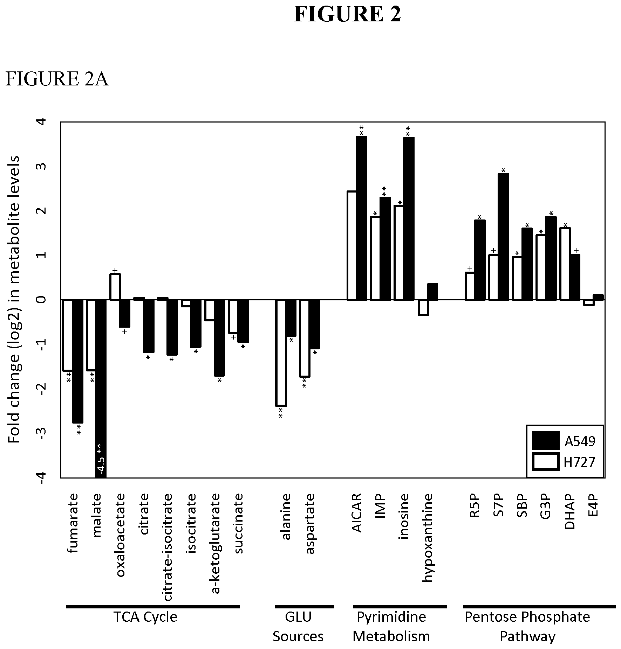

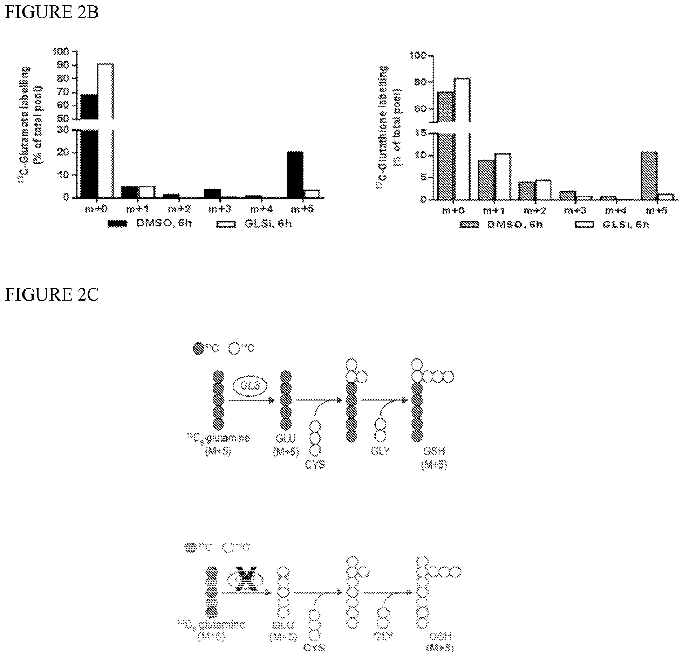

Figure 2 shows metabolic alterations in response to IACS-011393. Figure 2A shows the results of the metabolic analysis of A549 and H727 cells treated with 1 µM IACS-011393 for 24 hours. Figure 2B shows glutaminase inhibition by IACS-011393 prevents the conversion of glutamine to glutathione. Figure 2C shows a schematic representation of the incorporation of carbons from fully labeled 13C-Glutamine into glutathione in the absence or presence of the GLSi, IACS-011393.

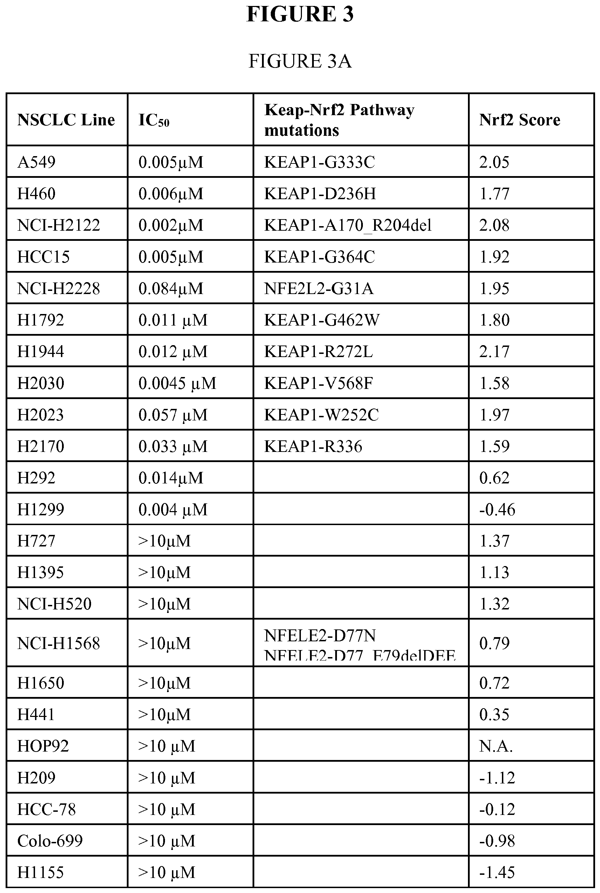

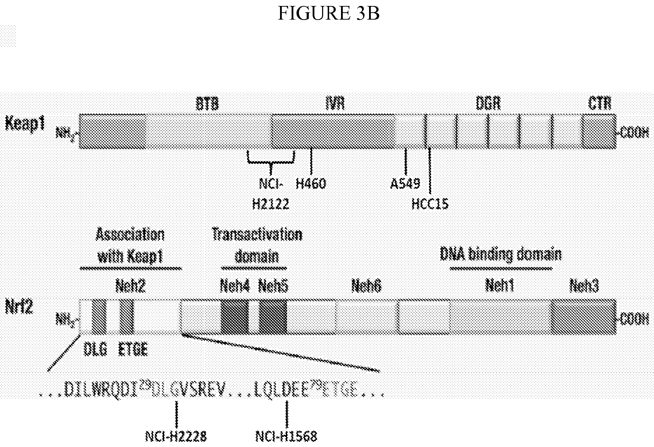

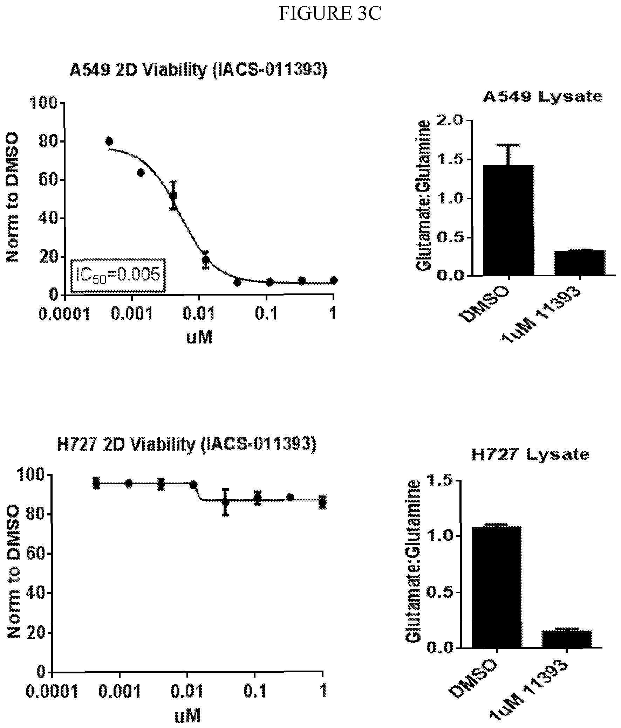

Figure 3 shows that mutations in the Keap1/Nrf2 pathway, a major regulator of redox balance, predict sensitivity to GLSi in pre-clinical models of NSCLC. Figure 3A shows the differential sensitivity to IACS-011393 is predicted by Keap1/Nrf2 status. Figure 3B shows that the mutations in responder cell lines fall within important functional domains of Keap1 and Nrf2. Figure 3C shows that target engagement is seen in both responder and non-responder cell lines, although responder cell lines are hyper-dependent on GLS-mediated glutaminolysis, while non-responder lines are less dependent on this process.

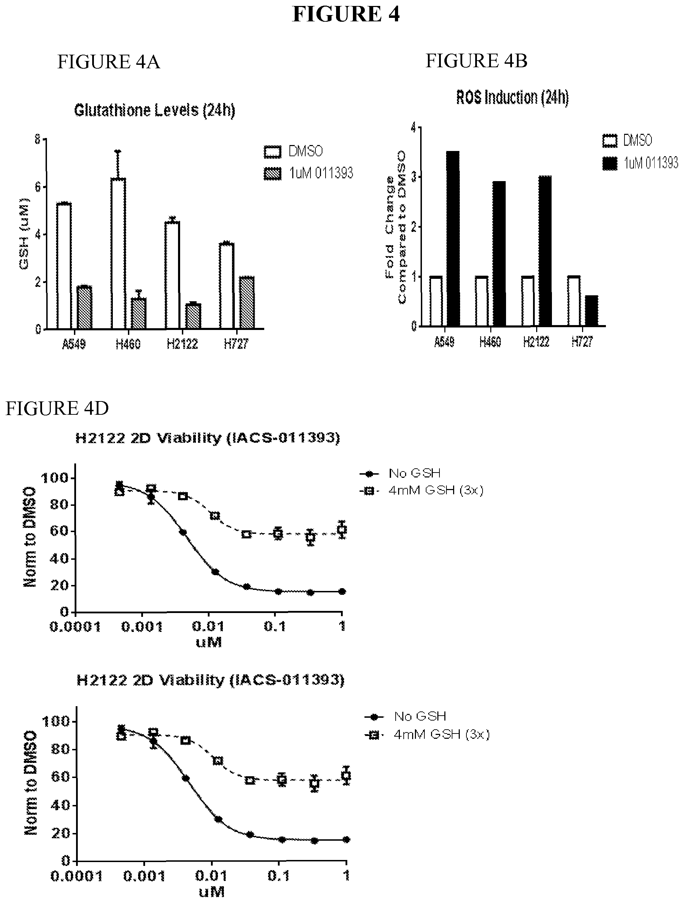

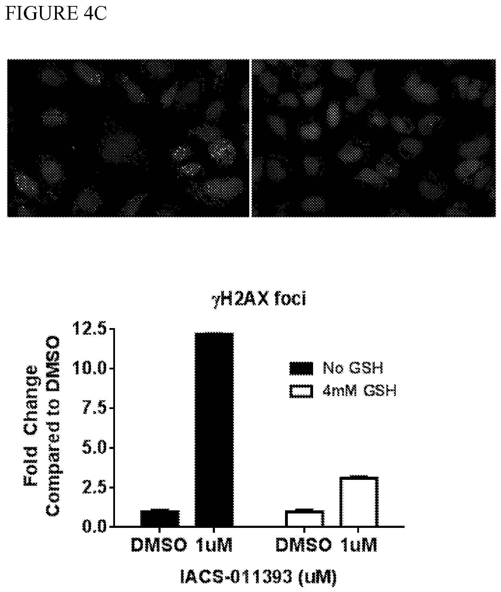

Figure 4 shows that GLS-dependence is driven by addiction to glutathione-mediated redox maintenance in Keap1/Nrf2 mutant cell lines. Figure 4A shows that glutathione levels are decreased after treatment with IACS-011393 in responder cell lines. Figure 4B shows the loss of glutathione after treatment with a glutaminase inhibitor leads to an accumulation of intracellular reactive oxygen species(ROS). Figure 4C shows that accumulation of DNA damage after IACS-011393 treatment can be rescued by application of cell-permeable glutathione. Figure 4D shows that application of exogenous glutathione to responder cell lines rescues IACS-011393-induced proliferation defects.

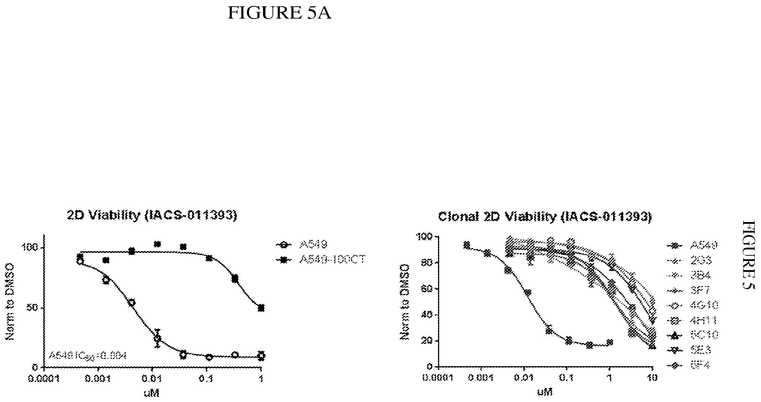

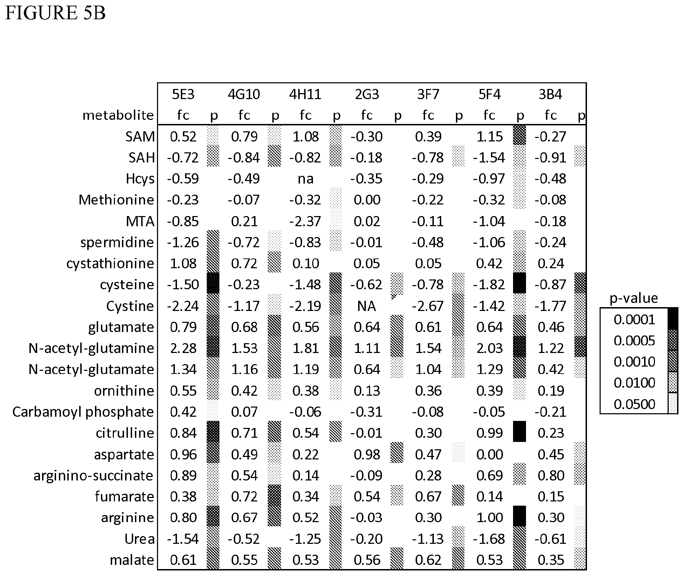

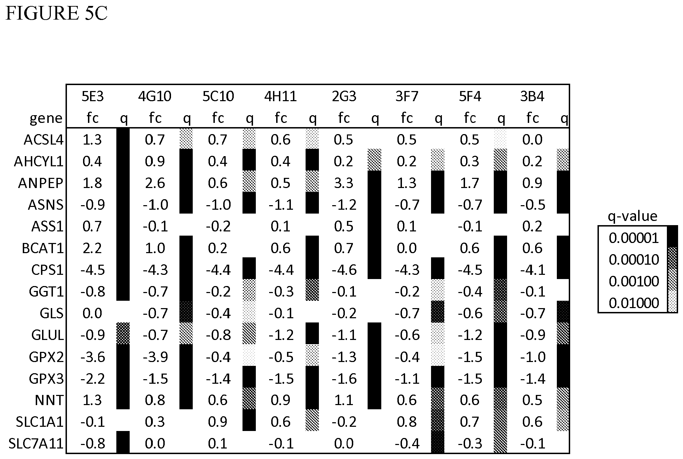

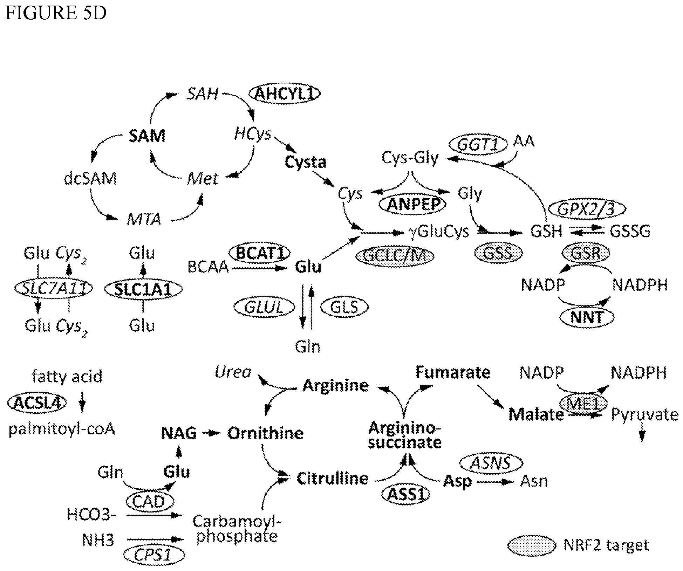

Figure 5 shows that acquired resistance to IACS-011393 is derived when cells activate alternative mechanisms to maintain redox balance. Figure 5A shows that cells treated chronically with GLSi acquire resistance to IACS-011393. Figure 5B shows that clones resistant to GLSi display activation of alternative pathways to maintain redox balance. Figure 5C shows that clones resistant to GLSi display altered transcriptional profiles to produce alternative sources of glutamate and reducing power. Figure 5D shows a global overview of acquired adaptation to glutaminase inhibition.

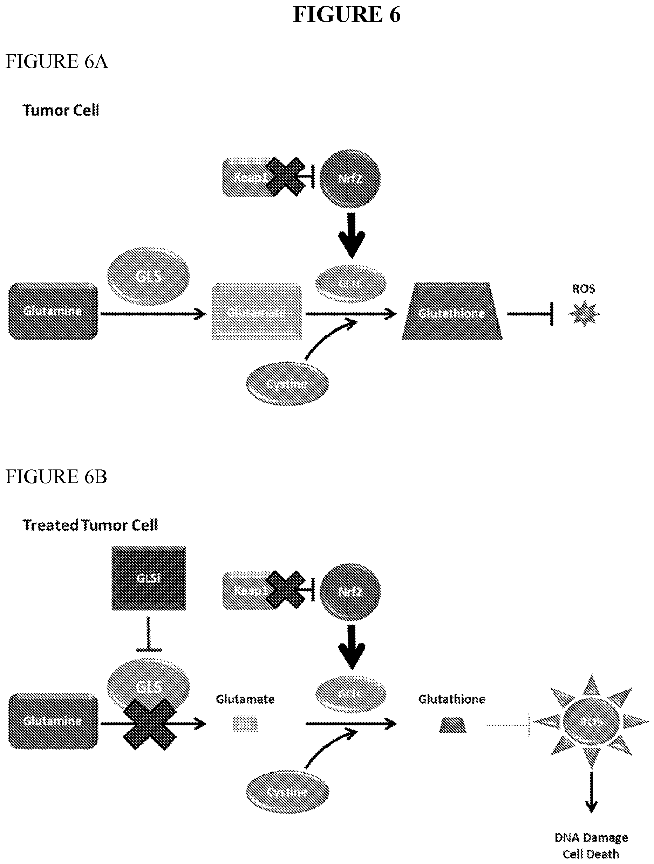

Figure 6 shows that NRF2 hyper-activation drives dependence on GLS-mediated glutathione synthesis to maintain redox balance.

### DETAILED DESCRIPTION

The invention is defined by the claims. Any subject-matter falling outside the scope of the claims is provided for information only. The present invention relates to a glutaminase inhibitor for use in the treatment of lung cancer, wherein the NRF2/KEAP1 pathway in the cancer cells is deregulated, and wherein the glutaminase inhibitor is selected from:
N,N'-(5,5'-(2,2'-sulfonylbis(ethane-2,1-diyl))bis(1,3,4-thiadiazole-5,2-diyl))bis(2-(pyridin-2-yl)acetamide),
N-methyl-1-{4-[6-(2-{4-[3-(trifluoromethoxy)phenyl]pyridin-2-yl}acetamido)pyridazin-3-yl]butyl}-1H-1,2,3-triazole-4-carboxamide,
1-(2-fluoro-4-(5-(2-(pyridin-2-yl)acetamido)-1,3,4-thiadiazol-2-yl)butyl)-N-((4-(trifluoromethyl)pyridin-2-yl)methyl)-1H-1,2,3-triazole-4-carboxamide,
1-(2-fluoro-4-(6-(2-(4-(trifluoromethyl)pyridin-2-yl)acetamido)pyridazin-3-yl)butyl)-N-methyl-1H-1,2,3-triazole-4-carboxamide,
N-(pyridin-2-ylmethyl)-5-(3-(6-(2-(3-(trifluoromethoxy)phenyl)acetamido)pyridazin-3-yl)pyrrolidin-1-yl)-1,3,4-thiadiazole-2-carboxamide,
(R)-1-(2-fluoro-4-(6-(2-(4-(3-(trifluoromethoxy)phenyl)pyridin-2-yl)acetamido)pyridazin-3-yl)butyl)-N-methyl-1H-1,2,3-triazole-4-carboxamide,
(R)-1-(2-fluoro-4-(6-(2-(4-(trifluoromethyl)pyridin-2-yl)acetamido)pyridazin-3-yl)butyl)-N-methyl-1H-1,2,3-triazole-4-carboxamide,
(R)-1-(2-fluoro-4-(6-(2-(6-methyl-4-(trifluoromethyl)pyridin-2-yl)acetamido)pyridazin-3-yl)butyl)-N-methyl-1H-1,2,3-triazole-4-carboxamide,
(R)-1-(4-(6-(2-(4-(cyclopropyldifluoromethyl)pyridin-2-yl)acetamido)pyridazin-3-yl)-2-fluorobutyl)-N-methyl-1H-1,2,3-triazole-4-carboxamide,
(R)-1-(4-(6-(2-(4-(3,3-difluorocyclobutoxy)pyridin-2-yl)acetamido)pyridazin-3-yl)-2-fluorobutyl)-N-methyl-1H-1,2,3-triazole-4-carboxamide,
(R)-1-(2-fluoro-4-(6-(2-(1-(3-(trifluoromethoxy)phenyl)-1H-imidazol-4-yl)acetamido)pyridazin-3-yl)butyl)-N-methyl-1H-1,2,3-triazole-4-carboxamide,
1-(4-(6-(2-(4-cyclobutoxypyridin-2-yl)acetamido)pyridazin-3-yl)butyl)-N-methyl-1H-1,2,3-triazole-4-carboxamide,
1-(4-(6-(2-(4-cyclobutoxypyridin-2-yl)acetamido)pyridazin-3-yl)-2-fluorobutyl)-N-methyl-1H-1,2,3-triazole-4-carboxamide,
1-(4-(6-(2-(4-(3,3-difluorocyclobutoxy)pyridin-2-yl)acetamido)pyridazin-3-yl)butyl)-N-methyl-1H-1,2,3-triazole-4-carboxamide,
1-(4-(6-(2-(4-(3,3-difluorocyclobutoxy)pyridin-2-yl)acetamido)pyridazin-3-yl)-2-fluorobutyl)-N-methyl-1H-1,2,3-triazole-4-carboxamide,
(R)-1-(4-(6-(2-(4-cyclopropylpyridin-2-yl)acetamido)pyridazin-3-yl)-2-fluorobutyl)-N-methyl-1H-1,2,3-triazole-4-carboxamide,
5-(3-(6-(2-(pyridin-2-yl)acetamido)pyridazin-3-yl)pyrrolidin-1-yl)-N-((4-(trifluoromethyl)pyridin-2-yl)methyl)-1,3,4-thiadiazole-2-carboxamide, and
N,N'-(5,5'-(cyclohexane-1,3-diyl)bis(1,3,4-thiadiazole-5,2-diyl))bis(2-phenylacetamide), or a salt thereof.

The invention is based on the discovery of biomarkers and mechanisms associated with the responsiveness to treatment with glutaminase (GLS) inhibitors and methods to treat such patients. In particular, alterations to the NRF2/KEAP1 pathway sensitize patients to GLS inhibition. KEAP1, a ubiquitin ligase adaptor protein, is a negative regulator of NRF2, the key transcription factor that activates the cellular antioxidant response. Deregulation of the NRF2/KEAP1 pathway, NRF2 signaling, an increase in the intracellular concentration of glutathione, or mutations in KEAP1 or NRF2 (for example, a loss-of-function mutation in KEAP1, or a gain-of-function mutation in NRF2) confer a dependence of tumors on reduced glutathione, the major endogenous antioxidant comprised of glycine, cysteine, and glutamine-derived glutamate. Inhibition of GLS reduces the steady state levels of glutathione, thus shifting the redox balance of tumor cells.

Nuclear factor (erythroid-derived 2)-like 2, also known as NRF2, is a transcription factor that in humans is encoded by the NFE2L2 gene. NRF2 is ubiquitously expressed at low levels in all human organs. The NRF2 antioxidant response pathway is the primary cellular defense against the cytotoxic effects of oxidative stress. Among other effects, NRF2 increases the expression of several antioxidant enzymes. As NRF2 regulates a major cellular defense mechanism, tight regulation is crucial to maintain cellular homeostasis. Activation of this pathway is important in preventing human diseases, such as cancer, neurodegenerative disease, cardiovascular diseases, ischemia, diabetes, pulmonary fibrosis, and inflammatory diseases. Conversely, high constitutive levels of NRF2 occur in many tumors or cancer cell lines. Moreover, overexpression of NRF2 in cancer cells protects them from the cytotoxic effects of anticancer therapies, resulting in chemo- and/or radioresistance.

Under normal or unstressed conditions, NRF2 is kept in the cytoplasm by a cluster of proteins that degrade it quickly. Under oxidative stress, NRF2 is not degraded, but instead travels to the nucleus where it binds to a DNA promoter and initiates transcription of antioxidative genes and their proteins. NRF2 is kept in the cytoplasm by Kelch like-ECH-associated protein 1 (KEAP1) and Cullin 3 which degrade NRF2 by ubiquitination. Cullin 3 ubiquitinates its substrate, NRF2. KEAP1 is a substrate adaptor, which helps Cullin 3 ubiquitinate NRF2. When NRF2 is ubiquitinated, it is transported to the proteasome, where it is degraded and its components recycled. Under normal conditions NRF2 has a half-life of only 20 minutes. Oxidative stress or electrophilic stress disrupts critical cysteine residues in KEAP1, disrupting the KEAP1-Cul3 ubiquitination system. When NRF2 is not ubiquitinated, it builds up in the cytoplasm, and translocates into the nucleus. In the nucleus, it combines (forms a heterodimer) with a small Maf protein and binds to the Antioxidant Response Element (ARE) in the upstream promoter region of many antioxidative genes, and initiates their transcription.

The NRF2/KEAP1 pathway is the major regulator of cytoprotective responses to endogenous and exogenous stresses caused by reactive oxygen species (ROS) and electrophiles. The key signaling proteins within the pathway are the transcription factor NRF2 that binds together with small Maf proteins to the antioxidant response element (ARE) in the regulatory regions of target genes, and KEAP1, the repressor protein that binds to NRF2 and promotes its degradation by the ubiquitin proteasome pathway. KEAP1 is a very cysteine-rich protein, mouse KEAP1 having a total of 25 and human 27 cysteine residues, most of which can be modified *in vitro* by different oxidants and electrophiles. Three of these residues, C151, C273 and C288, have been shown to play a functional role by altering the conformation of KEAP1 leading to nuclear translocation of NRF2 and subsequent target gene expression.

Consistent with this model, treatment of cancer cell lines having an altered NRF2/KEAP1 pathway with a potent GLS inhibitor reduced intracellular levels of glutamate and glutathione and inhibited cell growth. Without being bound by any theory, the inventors propose that the inhibition of cell growth is through a mechanism that involves the accumulation of reactive oxygen species (ROS)-induced DNA damage. In contrast, cancer cell lines without any of the above-stated conditions, for example, those that were wild type for NRF2/KEAP1 failed to respond to GLS inhibitor treatment, confirming the sensitivity of this biomarker and mechanism to predict response in the clinic.

Accordingly, in one aspect which is not part of the present invention, the disclosure comprises a method of treating a subject having a disorder in need of treatment. The method comprises (a) determining: (i) that the NRF2/KEAP1 pathway in said subject is deregulated, (ii) that NRF2 signaling in said subject is hyperactive, (iii) that the nucleic acid of said subject comprises a loss-of-function mutation in KEAP1, (iv) that the nucleic acid of said subject comprises a gain-of-function mutation in NRF2; or (v) an increase in the intracellular concentration of glutathione in said subject; and (b) administering a glutaminase inhibitor to the subject.

In one aspect which is not part of the present invention, disclosed herein is a method of treating a subject having a disorder in need of treatment comprising determining that the subject has an altered NRF2/KEAP1 pathway, and administering a glutaminase inhibitor to the subject. In another aspect which is not part of the present invention, the disclosure comprises a method of treating a subject having a disorder in need of treatment by administering a glutaminase inhibitor to the subject, wherein the subject has an altered NRF2/KEAP1 pathway.

The NRF2/KEAP1 pathway may be altered in one of several different mechanisms. In one embodiment, the NRF2/KEAP1 pathway in said subject is deregulated and ubiquitination of NRF2 which ultimately leads to its degradation is prevented. In certain embodiments the NRF2/KEAP1 pathway may be deregulated due to a mutation in either NRF2 or KEAP1. The mutation may be any mutation that disrupts the function of KEAP1 or a mutation in either KEAP1 or NRF2 prevents the binding of KEAP1 to NRF2. In one embodiment, the mutation is a somatic mutation.

In another embodiment, the NRF2/KEAP1 pathway may be deregulated due to epigenetic silencing of KEAP1 expression. Epigenetic silencing of KEAP1 expression results in constitutively activated NRF2 signaling. In one embodiment, DNA hypermethylation at the promoter region of KEAP1 results in epigenetic silencing of KEAP1 expression. Subjects with epigenetic silencing of KEAP1 expression are good candidates for treatment with glutaminase inhibitor.

In yet another embodiment, he NRF2/KEAP1 pathway may be deregulated due to accumulation of disruptor proteins leading to dissociation of the KEAP1-NRF2 complex. Examples of disruptor proteins include, but are not limited to, cyclin-dependent kinase inhibitor, p21, and polyubiquitin binding protein, p62.

Transcriptional induction of NRF2 is another mechanism by which the NRF2/KEAP1 pathway would be deregulated. The transcriptional induction of NRF2 may, for example, be caused by oncogenic signaling due to elements including, but not limited to, K-Ras, B-Raf and c-Myc.

In another embodiment, the NRF2/KEAP1 pathway may be deregulated due to post-translational modification of KEAP1 that affects binding of KEAP1 to NRF2. Any of the amino acid residues of KEAP1 may undergo post-translational modification. In one embodiment, the cysteine residues of KEAP1 are modified. Post-translational modification of KEAP1, for example modification of the cysteine residues, includes, but is not limited to, succination of critical cysteine residues. Critical KEAP1 cysteine residues include, but are not limited to, those at positions 151, 273 and 288.

In another aspect which is not part of the present invention, disclosed herein is a method of treating a subject having a disorder in need of treatment comprising determining that NRF2 signaling in said subject is hyperactive and administering a glutaminase inhibitor to the subject. As described above, NRF2 signaling could be hyperactive due to several different factors, such as disruption of KEAP1-NRF2 binding or a reduction or loss in the function of KEAP1, caused, for example, by any of the mechanisms described above. NRF2 signaling could also be hyperactive due to factors that affect expression of NRF2, including induction of NRF2 by oncogenic signaling or mutations in NRF2 that result in increasing its expression or function.

In certain embodiments, the nucleic acid of the subject having a disorder in need of treatment comprises a mutation in one of the proteins involved in the NRF2/KEAP1 pathway. In one embodiment, the mutation is a somatic mutation. In some embodiments, the nucleic acid encoding the KEAP1 protein comprises a mutation, for example, a loss-of-function mutation, or a mutation that results in the reduction or the loss of the KEAP1 protein's ability to bind NRF2. In other embodiments, the nucleic acid encoding the NRF2 protein comprises a mutation, for example, a gain-of-function mutation, or a mutation that results in the reduction or the loss of the NRF2 protein's ability to bind KEAP1 or any other protein involved in the ubiquitination of NRF2.

In yet another aspect which is not part of the present invention, disclosed herein is a method of treating a subject having a disorder in need of treatment comprising determining that an increase in the intracellular concentration of glutathione in said subject and administering a glutaminase inhibitor to the subject. Several mechanisms exist by which the intracellular concentration of glutathione (GSH) in a subject may be raised. Examples of such mechanisms include deregulated NRF2 activity or a deregulated NRF2/KEAP1 pathway as described above. The intracellular concentration of glutathione in a subject may be increased due to increased expression or activity of glutathione-related enzymes. Examples of such enzymes include γ-glutamylcysteine ligase and γ-glutamyl-transpeptidase. The intracellular concentration of glutathione may be increased due to increased expression or activity of amino acid transporters.

In another embodiment of the invention, the subject has: a deregulated NRF2/KEAP1 pathway; hyperactive NRF2 signaling; a loss-of-function mutation in the KEAP1 nucleic acid; a gain-of-function mutation in NRF2 nucleic acid; or an increase in intracellular concentration of glutathione. In certain embodiments, the subject has a deregulated NRF2/KEAP1 pathway. In certain embodiments, the subject has hyperactive NRF2 signaling. In certain embodiments, the subject has a loss-of-function mutation in the KEAP1 nucleic acid. In certain embodiments, the subject has a gain-of-function mutation in NRF2 nucleic acid. In certain embodiments, the subject has an increase in intracellular concentration of glutathione. In certain embodiments, the subject has more than one of the foregoing.

In another aspect which is not part of the present invention, the disclosure comprises a method of treating a subject having a disorder in need of treatment by administering a compound that inhibits glutathione production to a subject. The method comprises optionally obtaining a biological sample from a subject, determining that the NRF2/KEAP1 pathway in said subject is deregulated, or that NRF2 signaling in said subject is hyperactive, or determining the presence of a loss-of-function mutation in KEAP1 or a gain-of-function mutation in NRF2 of said subject, or more than one of the foregoing, and administering a compound that inhibits glutathione production. The compound may inhibit amino acid transport or glutathione synthesis. The compound that inhibits glutathione production may be a glutaminase inhibitor. The subject may have a deregulated NRF2/KEAP1 pathway. The subject may have hyperactive NRF2 signaling. The subject may have a loss-of-function mutation in the KEAP1 nucleic acid. The subject may have a gain-of-function mutation in NRF2 nucleic acid. The subject may have an increase in intracellular concentration of glutathione. The subject may have more than one of the foregoing.

In another aspect which is not part of the present invention, the disclosure comprises a method of evaluating whether to administer a compound that inhibits glutathione production to a subject. The method comprises optionally obtaining a biological sample from a subject, determining that the NRF2/KEAP1 pathway in said subject is deregulated, or that NRF2 signaling in said subject is hyperactive, or determining the presence of a loss-of-function mutation in KEAP1 or a gain-of-function mutation in NRF2 of said subject and determining that the compound that inhibits glutathione production should be administered to the subject. The compound may inhibit amino acid transport or glutathione synthesis. The subject may have a deregulated NRF2/KEAP1 pathway The subject may have hyperactive NRF2 signaling. The subject may have a loss-of-function mutation in the KEAP1 nucleic acid. The subject may have a gain-of-function mutation in NRF2 nucleic acid. The subject may have an increase in intracellular concentration of glutathione. The subject may have more than one of the foregoing.

Also provided herein is a glutaminase inhibitor or a compound that inhibits glutathione production as defined in the claims for use as a medicament in the treatment of lung cancer, in a subject in which (i) the NRF2/KEAP1 pathway in said subject is deregulated, (ii) NRF2 signaling in said subject is hyperactive, (iii) that the nucleic acid of said subject comprises a loss-of-function mutation in KEAP1, (iv) the nucleic acid of said subject comprises a gain-of-function mutation in NRF2; or (v) the subject has an increase in the intracellular concentration of glutathione.

Also provided herein is a glutaminase inhibitor or a compound that inhibits glutathione production as defined in the claims for use in the treatment of lung cancer in a subject in which (i) the NRF2/KEAP1 pathway in said subject is deregulated, (ii) NRF2 signaling in said subject is hyperactive, (iii) that the nucleic acid of said subject comprises a loss-of-function mutation in KEAP1, (iv) the nucleic acid of said subject comprises a gain-of-function mutation in NRF2; or (v) the subject has an increase in the intracellular concentration of glutathione.

In certain embodiments, the NRF2/KEAP1 pathway in said subject is deregulated because of:
(a) a somatic mutation present in KEAP1 or NRF2;
(b) epigenetic silencing of KEAP1 expression;
(c) accumulation of disruptor proteins leading to dissociation of the KEAP1/NRF2 complex;
(d) transcriptional induction of NRF2 by oncogenic signaling;
(e) post-translational modification of KEAP1 that affects binding to NRF2; or
(f) expression of a microRNA that down-regulates KEAP1 levels.

In certain embodiments, the NRF2/KEAP1 pathway in said subject is deregulated because of a somatic mutation in KEAP1 or the KEAP1 binding domain of NRF2. In certain embodiments, the NRF2/KEAP1 pathway in said subject is deregulated because of DNA hypermethylation at the promoter region of KEAP1. In certain embodiments, the NRF2/KEAP1 pathway in said subject is deregulated because of accumulation of cyclin-dependent kinase inhibitor p21 or polyubiquitin binding protein p62. In certain embodiments, the NRF2/KEAP1 pathway in said subject is deregulated because of transcriptional induction of NRF2 by K-Ras, B-Raf or c-Myc oncogenic signaling. In certain embodiments, the NRF2/KEAP1 pathway in said subject is deregulated because of post-translational modification of KEAP1 cysteine residues. In certain embodiments, the NRF2/KEAP1 pathway in said subject is deregulated because of expression of a microRNA that down-regulates KEAP1 levels.

The subject may have an increase in the intracellular concentration of glutathione (GSH), which is caused by:
(a) deregulated NRF2 activity;
(b) increased expression of GSH-related enzymes; or
(c) increased expression or activity of amino acid transporters.

The increase in the intracellular concentration of glutathione (GSH) in said subject may be caused by deregulated NRF2 activity. The increase in the intracellular concentration of glutathione (GSH) in said subject may be caused by increased expression of GSH-related enzymes. The increase in the intracellular concentration of glutathione (GSH) in said subject may be caused by increased expression or activity of amino acid transporters.

In certain embodiments, the cancer is non-small cell lung cancer (NSCLC).

The glutaminase inhibitor according to the invention is a selective inhibitor of GLS-1.

In certain embodiments, the GLS-1 inhibitor binds an allosteric pocket on the solvent exposed region of the GLS-1 dimer in the binding pocket present in the vicinity of Leu321, Phe322, Leu323, and Tyr394 from both monomers.

The glutaminase inhibitor is chosen from the list of compounds provided in Table 1 below. In certain embodiments, the compound is chosen from any combination of the compounds provided in Table 1, or a salt or polymorph thereof. For example, in certain embodiments, the compound is chosen from any two, three, four, five, six, seven, eight, none or ten of the compounds provided in Table 1, or a salt or polymorph thereof.

In certain embodiments, the subject is human.

In certain embodiments, treatments disclosed herein further comprise administering another pharmaceutically active compound. In certain embodiments, the other pharmaceutically active compound is an anti-cancer agent. In certain embodiments, the anti-cancer agent is a chosen from a platinum-based agent, a taxane-based agent, an immunotherapy, a targeted therapy. In certain embodiments, the targeted therapy is an inhibitor of MEK kinase, HSP90, CDK4, or the mTOR pathway.

In certain embodiments, treatments disclosed herein further comprise administering non-chemical methods of cancer treatment. In certain embodiments, the treatment further comprises administering radiation therapy. In certain embodiments, the treatment further comprises administering surgery, thermoablation, focused ultrasound therapy, cryotherapy, or any combination thereof.

In certain embodiments, treatments disclosed herein administer the active agent (i.e., the compound that inhibits glutathione production, glutaminase inhibitor, or selective GLS-1 inhibitor according to the claims) as a pharmaceutical composition comprising a pharmaceutically acceptable excipient or carrier. In certain embodiments, the pharmaceutical composition is formulated for oral administration. In certain embodiments, pharmaceutical composition is formulated as a tablet or capsule. In certain embodiments, the pharmaceutical composition is formulated for parenteral administration.

### Abbreviations and Definitions

To facilitate understanding of the disclosure, a number of terms and abbreviations as used herein are defined below as follows:

When introducing elements of the present disclosure or the preferred embodiment(s) thereof, the articles "a," "an," "the" and "said" are intended to mean that there are one or more of the elements. The terms "comprising," "including" and "having" are intended to be inclusive and mean that there may be additional elements other than the listed elements.

The term "and/or" when used in a list of two or more items, means that any one of the listed items can be employed by itself or in combination with any one or more of the listed items. For example, the expression "A and/or B" is intended to mean either or both of A and B, *i.e.,* A alone, B alone or A and B in combination. The expression "A, B and/or C" is intended to mean A alone, B alone, C alone, A and B in combination, A and C in combination, B and C in combination or A, B, and C in combination.

When ranges of values are disclosed, and the notation "from n1 ... to n2" or "between n1 ... and n2" is used, where n1 and n2 are the numbers, then unless otherwise specified, this notation is intended to include the numbers themselves and the range between them. This range may be integral or continuous between and including the end values. By way of example, the range "from 2 to 6 carbons" is intended to include two, three, four, five, and six carbons, since carbons come in integer units. Compare, by way of example, the range "from 1 to 3 µM (micromolar)," which is intended to include 1 µM, 3 µM, and everything in between to any number of significant figures (e.g., 1.255 µM, 2.1 µM, 2.9999 µM, etc.).

The term "about," as used herein in relation to a numerical value x means x ± 10%.

The term "disease" as used herein is intended to be generally synonymous, and is used interchangeably with, the terms "disorder," "syndrome," and "condition" (as in medical condition), in that all reflect an abnormal condition of the human or animal body or of one of its parts that impairs normal functioning, is typically manifested by distinguishing signs and symptoms, and causes the human or animal to have a reduced duration or quality of life.

The term "combination therapy" means the administration of two or more therapeutic agents to treat a therapeutic condition or disorder described in the present disclosure. Such administration encompasses co-administration of these therapeutic agents in a substantially simultaneous manner, such as in a single capsule having a fixed ratio of active ingredients or in multiple, separate capsules for each active ingredient. In addition, such administration also encompasses use of each type of therapeutic agent in a sequential manner. In either case, the treatment regimen will provide beneficial effects of the drug combination in treating the conditions or disorders described herein.

The terms "altered NRF2/KEAP1 pathway" or a "NRF2/KEAP1 pathway that is altered" are used interchangeably herein and refer to a subject or a cell or a cell line in which any of the following are present: (i) a deregulated NRF2/KEAP1 pathway; (ii) hyperactive NRF2 signaling; (iii) a loss-of-function mutation in KEAP1; (iv) a gain-of-function mutation in NRF2; or (v) an increase in the intracellular concentration of glutathione.

GLS-1 inhibitor is used herein to refer to a compound that exhibits an IC₅₀ with respect to GLS-1 activity of no more than about 100 µM and more typically not more than about 50 µM, as measured in the GLS-1 enzyme assay described generally herein below. IC₅₀ is that concentration of inhibitor that reduces the activity of an enzyme (e.g., GLS-1) to half-maximal level. Certain compounds disclosed herein have been discovered to exhibit inhibition against GLS-1. In certain embodiments, compounds will exhibit an IC₅₀ with respect to GLS-1 of no more than about 10 µM; in further embodiments, compounds will exhibit an IC₅₀ with respect to GLS-1 of no more than about 5 µM; in yet further embodiments, compounds will exhibit an IC₅₀ with respect to GLS-1 of not more than about 1 µM; in yet further embodiments, compounds will exhibit an IC₅₀ with respect to GLS-1 of not more than about 200 nM, as measured in the GLS-1 enzymatic assay described herein.

The terms "inhibitor selective for GLS-1" or a "selective inhibitor of GLS-1" are used interchangeably herein and refer to inhibitors that are about 100 times more selective for GLS-1 than for GLS-2 as measured in any assay known to one of skill in the art that measures the activity of the enzyme. An example of such an assay includes, but is not limited to, the GLS-1 enzyme assay (GLS-1 Enzymatic Activity Assay) described below.

The phrase "therapeutically effective" is intended to qualify the amount of active ingredients used in the treatment of a disease or disorder or on the effecting of a clinical endpoint.

The term "therapeutically acceptable" refers to those compounds (or salts, prodrugs, tautomers, zwitterionic forms, etc.) which are suitable for use in contact with the tissues of patients without undue toxicity, irritation, and allergic response, are commensurate with a reasonable benefit/risk ratio, and are effective for their intended use.

As used herein, reference to "treatment" of a patient is intended to include prevention, prophylaxis, attenuation, amelioration and therapy. Treatment may also include prevention of disease. Prevention of a disease may involve complete protection from disease, for example as in the case of prevention of infection with a pathogen, or may involve prevention of disease progression. For example, prevention of a disease may not mean complete foreclosure of any effect related to the diseases at any level, but instead may mean prevention of the symptoms of a disease to a clinically significant or detectable level. Prevention of diseases may also mean prevention of progression of a disease to a later stage of the disease.

The terms "subject" or "patient" are used interchangeably herein to mean all mammals including humans. Examples of subjects include, but are not limited to, humans, monkeys, dogs, cats, horses, cows, goats, sheep, pigs, and rabbits. In one embodiment, the patient is a human.

The terms "affected with a disease or disorder," "afflicted with a disease or disorder," or "having a disease or disorder" are used interchangeably herein and refer to a subject or patient with any disease, disorder, syndrome or condition. No increased or decreased level of severity of the disorder is implied by the use of one the terms as compared to the other.

The compounds disclosed herein can exist as therapeutically acceptable salts. The present disclosure includes compounds listed above in the form of salts, including acid addition salts. Suitable salts include those formed with both organic and inorganic acids. Such acid addition salts will normally be pharmaceutically acceptable. However, salts of non-pharmaceutically acceptable salts may be of utility in the preparation and purification of the compound in question. Basic addition salts may also be formed and be pharmaceutically acceptable. For a more complete discussion of the preparation and selection of salts, refer to Pharmaceutical Salts: Properties, Selection, and Use (Stahl, P. Heinrich. Wiley-VCHA, Zurich, Switzerland, 2002).

The term "therapeutically acceptable salt," as used herein, represents salts or zwitterionic forms of the compounds disclosed herein which are water or oil-soluble or dispersible and therapeutically acceptable as defined herein. The salts can be prepared during the final isolation and purification of the compounds or separately by reacting the appropriate compound in the form of the free base with a suitable acid. Representative acid addition salts include acetate, adipate, alginate, L-ascorbate, aspartate, benzoate, benzenesulfonate (besylate), bisulfate, butyrate, camphorate, camphorsulfonate, citrate, digluconate, formate, fumarate, gentisate, glutarate, glycerophosphate, glycolate, hemisulfate, heptanoate, hexanoate, hippurate, hydrochloride, hydrobromide, hydroiodide, 2-hydroxyethansulfonate (isethionate), lactate, maleate, malonate, DL-mandelate, mesitylenesulfonate, methanesulfonate, naphthylenesulfonate, nicotinate, 2-naphthalenesulfonate, oxalate, pamoate, pectinate, persulfate, 3-phenylproprionate, phosphonate, picrate, pivalate, propionate, pyroglutamate, succinate, sulfonate, tartrate, L-tartrate, trichloroacetate, trifluoroacetate, phosphate, glutamate, bicarbonate, para-toluenesulfonate (p-tosylate), and undecanoate. Also, basic groups in the compounds disclosed herein can be quaternized with methyl, ethyl, propyl, and butyl chlorides, bromides, and iodides; dimethyl, diethyl, dibutyl, and diamyl sulfates; decyl, lauryl, myristyl, and steryl chlorides, bromides, and iodides; and benzyl and phenethyl bromides. Examples of acids which can be employed to form therapeutically acceptable addition salts include inorganic acids such as hydrochloric, hydrobromic, sulfuric, and phosphoric, and organic acids such as oxalic, maleic, succinic, and citric. Salts can also be formed by coordination of the compounds with an alkali metal or alkaline earth ion. Hence, the present disclosure contemplates sodium, potassium, magnesium, and calcium salts of the compounds disclosed herein.

Basic addition salts can be prepared during the final isolation and purification of the compounds by reacting a carboxy group with a suitable base such as the hydroxide, carbonate, or bicarbonate of a metal cation or with ammonia or an organic primary, secondary, or tertiary amine. The cations of therapeutically acceptable salts include lithium, sodium, potassium, calcium, magnesium, and aluminum, as well as nontoxic quaternary amine cations such as ammonium, tetramethylammonium, tetraethylammonium, methylamine, dimethylamine, trimethylamine, triethylamine, diethylamine, ethylamine, tributylamine, pyridine, N,N-dimethylaniline, N-methylpiperidine, N-methylmorpholine, dicyclohexylamine, procaine, dibenzylamine, N,N-dibenzylphenethylamine, 1-ephenamine, and N,N'-dibenzylethylenediamine. Other representative organic amines useful for the formation of base addition salts include ethylenediamine, ethanolamine, diethanolamine, piperidine, and piperazine.

A salt of a compound can be made by reacting the appropriate compound in the form of the free base with the appropriate acid.

### Compounds

The compounds useful in the treatments of the invention are provided in Table 1 below. The compound is a compound that inhibits glutathione production or activity. The compound inhibits amino acid or glutathione transport. The compound is a glutaminase inhibitor.

The compound is a GLS-1 inhibitor, for example, a selective inhibitor of GLS-1. It is known that GLS-1 forms a tetramer (PNAS 2012, 109, 7705). The GLS-1 inhibitor occupies an allosteric pocket on the solvent exposed region between two GLS-1 dimers. The GLS-1 inhibitor may, for example, bind GLS-1 in an allosteric pocket on the solvent exposed region of the GLS-1 dimer in the binding pocket present in the vicinity of amino acids Leu 321, Phe322, Leu323, and Tyr394 from both monomers. Without being bound by any theory, the inventors propose that key interactions are made within a hydrophobic cluster that comprises Leu321, Phe322, Leu323, and Tyr394 from both monomers which forms the allosteric pocket. Binding of the glutaminase inhibitor, for example, a GLS-1 inhibitor, induces a dramatic conformational change near the catalytic site rendering the enzyme inactive.

Compounds which inhibit GLS-1 are known in the art and disclosed herein. The compounds useful for the treatment of lung cancer according to the present invention (and their molecular mass) are provided in Table 1 below, or a salt or polymorph thereof. In certain embodiments, the compound is chosen from any combination of the compounds provided in Table 1 below, or a salt or polymorph thereof. For example, in certain embodiments, the compound is chosen from any two, three, four, five, six, seven, eight, none or ten of the compounds provided in Table 1 below, or a salt or polymorph thereof.

**Table 1**

| **Compound** | **MS** |
|---|---|
| (S)-2-hydroxy-2-phenyl-N-(5-(4-(6-(2-(3-(trifluoromethoxy)phenyl)acetamido)pyridazin-3-yl)butyl)-1,3,4-thiadiazol-2-yl)acetamide, also referred to as IACS-011393 (Non-claimed disclosure - Reference Example Only) | 586 |
| N,N'(5,5'(2,2'thiobis(ethane-2,1-diyl))bis(1,3,4-thiadiazole-5,2-diyl))bis(2-phenylacetamide), also known as BPTES (Non-claimed disclosure - Reference Example Only) | 524 |
| 2-(pyridin-2-yl)-N-{5-[4-(6-{2-[3-(trifluoromethoxy)phenyl]acetamido}pyridazin-3-yl)butyl]-1,3,4-thiadiazol-2-yl}acetamide, also known as CB-839(Non-claimed disclosure - Reference Example Only) | 587 |
| N,N'-(5,5'-(2,2'-sulfonylbis(ethane-2,1-diyl))bis(1,3,4-thiadiazole-5,2-diyl))bis(2-(pyridin-2-yl)acetamide), also referred to as Compound 13 | 558 |
| N-methyl-1-{4-[6-(2-{4-[3-(trifluoromethoxy)phenyl]pyridin-2-yl}acetamido)pyridazin-3-yl]butyl}-1H-1,2,3-triazole-4-carboxamide | 554 |
| 1-(2-fluoro-4-(5-(2-(pyridin-2-yl)acetamido)-1,3,4-thiadiazol-2-yl)butyl)-N-((4-(trifluoromethyl)pyridin-2-yl)methyl)-1H-1,2,3-triazole-4-carboxamide 1-(2-fluoro-4-(6-(2-(4-(trifluoromethyl)pyridin-2-yl)acetamido)pyridazin-3-yl)butyl)-N-methyl-1H-1,2,3-triazole-4-carboxamide | 563 480 |
| N-(pyridin-2-ylmethyl)-5-(3-(6-(2-(3-(trifluoromethoxy)phenyl)acetamido)pyridazin-3-yl)pyrrolidin-1-yl)-1,3,4-thiadiazole-2-carboxamide | 584 |
| (R)-1-(2-fluoro-4-(6-(2-(4-(3-(trifluoromethoxy)phenyl)pyridin-2-yl)acetamido)pyridazin-3-yl)butyl)-N-methyl-1H-1,2,3-triazole-4-carboxamide | 572 |
| (R)-1-(2-fluoro-4-(6-(2-(4-(trifluoromethyl)pyridin-2-yl)acetamido)pyridazin-3-yl)butyl)-N-methyl-1H-1,2,3-triazole-4-carboxamide | 480 |
| (R)-1-(2-fluoro-4-(6-(2-(6-methyl-4-(trifluoromethyl)pyridin-2-yl)acetamido)pyridazin-3-yl)butyl)-N-methyl-1H-1,2,3-triazole-4-carboxamide | 494 |
| (R)-1-(4-(6-(2-(4-(cyclopropyldifluoromethyl)pyridin-2-yl)acetamido)pyridazin-3-yl)-2-fluorobutyl)-N-methyl-1H-1,2,3-triazole-4-carboxamide | 502 |
| (R)-1-(4-(6-(2-(4-(3,3-difluorocyclobutoxy)pyridin-2-yl)acetamido)pyridazin-3-yl)-2-fluorobutyl)-N-methyl-1H-1,2,3-triazole-4-carboxamide | 518 |
| (R)-1-(2-fluoro-4-(6-(2-(1-(3-(trifluoromethoxy)phenyl)-1H-imidazol-4-yl)acetamido)pyridazin-3-yl)butyl)-N-methyl-1H-1,2,3-triazole-4-carboxamide | 561 |
| 1-(4-(6-(2-(4-cyclobutoxypyridin-2-yl)acetamido)pyridazin-3-yl)butyl)-N-methyl-1H-1,2,3-triazole-4-carboxamide | 464 |
| 1-(4-(6-(2-(4-cyclobutoxypyridin-2-yl)acetamido)pyridazin-3-yl)-2-fluorobutyl)-N-methyl-1H-1,2,3-triazole-4-carboxamide | 482 |
| 1-(4-(6-(2-(4-(3,3-difluorocyclobutoxy)pyridin-2-yl)acetamido)pyridazin-3-yl)butyl)-N-methyl-1H-1,2,3-triazole-4-carboxamide | 500 |
| 1-(4-(6-(2-(4-(3,3-difluorocyclobutoxy)pyridin-2-yl)acetamido)pyridazin-3-yl)-2-fluorobutyl)-N-methyl-1H-1,2,3-triazole-4-carboxamide | 518 |
| (R)-1-(4-(6-(2-(4-cyclopropylpyridin-2-yl)acetamido)pyridazin-3-yl)-2-fluorobutyl)-N-methyl-1H-1,2,3-triazole-4-carboxamide | 452 |
| 5-(3-(6-(2-(pyridin-2-yl)acetamido)pyridazin-3-yl)pyrrolidin-1-yl)-N-((4-(trifluoromethyl)pyridin-2-yl)methyl)-1,3,4-thiadiazole-2-carboxamide | 569 |
| N,N'-(5,5'-(cyclohexane-1,3-diyl)bis(1,3,4-thiadiazole-5,2-diyl))bis(2-phenylacetamide) (both or either of *1S,3S* and *1R, 2R* enantiomers) | 518 |

In certain embodiments, the compound is N-methyl-1-{4-[6-(2-{4-[3-(trifluoromethoxy)phenyl]pyridin-2-yl}acetamido)pyridazin-3-yl]butyl}-1H-1,2,3-triazole-4-carboxamide, or a salt or polymorph thereof. In certain embodiments, the compound is 1-(2-fluoro-4-(5-(2-(pyridin-2-yl)acetamido)-1,3,4-thiadiazol-2-yl)butyl)-N-((4-(trifluoromethyl)pyridin-2-yl)methyl)-1H-1,2,3-triazole-4-carboxamide, or a salt or polymorph thereof. In certain embodiments, the compound is 1-(2-fluoro-4-(6-(2-(4-(trifluoromethyl)pyridin-2-yl)acetamido)pyridazin-3-yl)butyl)-N-methyl-1H-1,2,3-triazole-4-carboxamide, or a salt or polymorph thereof. In certain embodiments, the compound is N-(pyridin-2-ylmethyl)-5-(3-(6-(2-(3-(trifluoromethoxy)phenyl)acetamido)pyridazin-3-yl)pyrrolidin-1-yl)-1,3,4-thiadiazole-2-carboxamide, or a salt or polymorph thereof. In certain embodiments, the compound is (R)-1-(2-fluoro-4-(6-(2-(4-(3-(trifluoromethoxy)phenyl)pyridin-2-yl)acetamido)pyridazin-3-yl)butyl)-N-methyl-1H -1,2,3-triazole-4-carboxamide, or a salt or polymorph thereof. In certain embodiments, the compound is (R)-1-(2-fluoro-4-(6-(2-(4-(trifluoromethyl)pyridin-2-yl)acetamido)pyridazin-3-yl)butyl)-N-methyl-1H-1,2,3-triazole-4-carboxamide, or a salt or polymorph thereof. In certain embodiments, the compound is (R)-1-(2-fluoro-4-(6-(2-(6-methyl-4-(trifluoromethyl)pyridin-2-yl)acetamido)pyridazin-3-yl)butyl)-N-methyl-1H -1,2,3-triazole-4-carboxamide, or a salt or polymorph thereof. In certain embodiments, the compound is (R)-1-(4-(6-(2-(4-(cyclopropyldifluoromethyl)pyridin-2-yl)acetamido)pyridazin-3-yl)-2-fluorobutyl)-N-methyl-1H-1,2,3-triazole-4-carboxamide, or a salt or polymorph thereof. In certain embodiments, the compound is (R)-1-(4-(6-(2-(4-(3,3-difluorocyclobutoxy)pyridin-2-yl)acetamido)pyridazin-3-yl)-2-fluorobutyl)-N-methyl-1H-1,2,3-triazole-4-carboxamide, or a salt or polymorph thereof. In certain embodiments, the compound is (R)-1-(2-fluoro-4-(6-(2-(1-(3-(trifluoromethoxy)phenyl)-1H-imidazol-4-yl)acetamido)pyridazin-3-yl)butyl)-N-methyl-1H-1,2,3-triazole-4-carboxamide, or a salt or polymorph thereof. In certain embodiments, the compound is 1-(4-(6-(2-(4-cyclobutoxypyridin-2-yl)acetamido)pyridazin-3-yl)butyl)-N-methyl-1H -1,2,3-triazole-4-carboxamide, or a salt or polymorph thereof. In certain embodiments, the compound is 1-(4-(6-(2-(4-cyclobutoxypyridin-2-yl)acetamido)pyridazin-3-yl)-2-fluorobutyl)-N-methyl-1H-1,2,3-triazole-4-carboxamide, or a salt or polymorph thereof. In certain embodiments, the compound is 1-(4-(6-(2-(4-(3,3-difluorocyclobutoxy)pyridin-2-yl)acetamido)pyridazin-3-yl)butyl)-N-methyl-1H-1,2,3-triazole-4-carboxamide, or a salt or polymorph thereof. In certain embodiments, the compound is 1-(4-(6-(2-(4-(3,3-difluorocyclobutoxy)pyridin-2-yl)acetamido)pyridazin-3-yl)-2-fluorobutyl)-N-methyl-1H-1,2,3-triazole-4-carboxamide, or a salt or polymorph thereof. In certain embodiments, the compound is (R)-1-(4-(6-(2-(4-cyclopropylpyridin-2-yl)acetamido)pyridazin-3-yl)-2-fluorobutyl)-N-methyl-1H-1,2,3-triazole-4-carboxamide, or a salt or polymorph thereof. In certain embodiments, the compound is 5-(3-(6-(2-(pyridin-2-yl)acetamido)pyridazin-3-yl)pyrrolidin-1-yl)-N-((4-(trifluoromethyl)pyridin-2-yl)methyl)-1,3,4-thiadiazole-2-carboxamide, or a salt or polymorph thereof. In certain embodiments, the compound is N,N'-(5,5'-(cyclohexane-1,3-diyl)bis(1,3,4-thiadiazole-5,2-diyl))bis(2-phenylacetamide), or a salt or polymorph thereof. In certain embodiments, the compound is N,N'-(5,5'-((1S,3S)-cyclohexane-1,3-diyl)bis(1,3,4-thiadiazole-5,2-diyl))bis(2-phenylacetamide), or a salt or polymorph thereof. In certain embodiments, the compound is N,N'-(5,5'-((1R,3R)-cyclohexane-1,3-diyl)bis(1,3,4-thiadiazole-5,2-diyl))bis(2-phenylacetamide), or a salt or polymorph thereof.

### Pharmaceutical Compositions

While it may be possible for the compounds disclosed herein to be administered as the raw chemical, it is also possible to present them as a pharmaceutical formulation. Accordingly, provided herein are pharmaceutical formulations which comprise one or more of certain compounds disclosed herein, or one or more pharmaceutically acceptable salts, esters, prodrugs, amides, or solvates thereof, together with one or more pharmaceutically acceptable carriers thereof and optionally one or more other therapeutic ingredients. The carrier(s) must be "acceptable" in the sense of being compatible with the other ingredients of the formulation and not deleterious to the recipient thereof. Proper formulation is dependent upon the route of administration chosen. Any of the well-known techniques, carriers, and excipients may be used as suitable and as understood in the art; e.g., in Remington's Pharmaceutical Sciences. The pharmaceutical compositions disclosed herein may be manufactured in any manner known in the art, e.g., by means of conventional mixing, dissolving, granulating, dragee-making, levigating, emulsifying, encapsulating, entrapping or compression processes.

The formulations include those suitable for oral, parenteral (including subcutaneous, intradermal, intramuscular, intravenous, intraarticular, and intramedullary), intraperitoneal, transmucosal, transdermal, rectal and topical (including dermal, buccal, sublingual and intraocular) administration although the most suitable route may depend upon for example the condition and disorder of the recipient. The formulations may conveniently be presented in unit dosage form and may be prepared by any of the methods well known in the art of pharmacy. Typically, these methods include the step of bringing into association a compound of the subject disclosure or a pharmaceutically acceptable salt, ester, amide, prodrug or solvate thereof ("active ingredient") with the carrier which constitutes one or more accessory ingredients. In general, the formulations are prepared by uniformly and intimately bringing into association the active ingredient with liquid carriers or finely divided solid carriers or both and then, if necessary, shaping the product into the desired formulation.

### Routes of Administration

### Oral Administration

The compounds of the present invention may be administered orally, including swallowing, so the compound enters the gastrointestinal tract, or is absorbed into the blood stream directly from the mouth, including sublingual or buccal administration.

Suitable compositions for oral administration include solid formulations such as tablets, pills, cachets, lozenges and hard or soft capsules, which can contain liquids, gels, powders, or granules.

In a tablet or capsule dosage form the amount of drug present may be from about 0.05% to about 95% by weight, more typically from about 2% to about 50% by weight of the dosage form.

In addition, tablets or capsules may contain a disintegrant, comprising from about 0.5% to about 35% by weight, more typically from about 2% to about 25% of the dosage form. Examples of disintegrants include methyl cellulose, sodium or calcium carboxymethyl cellulose, croscarmellose sodium, polyvinylpyrrolidone, hydroxypropyl cellulose, starch and the like.

Suitable binders, for use in a tablet, include gelatin, polyethylene glycol, sugars, gums, starch, hydroxypropyl cellulose and the like. Suitable diluents, for use in a tablet, include mannitol, xylitol, lactose, dextrose, sucrose, sorbitol and starch.

Suitable surface active agents and glidants, for use in a tablet or capsule, may be present in amounts from about 0.1% to about 3% by weight, and include polysorbate 80, sodium dodecyl sulfate, talc and silicon dioxide.

Suitable lubricants, for use in a tablet or capsule, may be present in amounts from about 0.1% to about 5% by weight, and include calcium, zinc or magnesium stearate, sodium stearyl fumarate and the like.

Tablets may be made by compression or molding, optionally with one or more accessory ingredients. Compressed tablets may be prepared by compressing in a suitable machine the active ingredient in a free-flowing form such as a powder or granules, optionally mixed with binders, inert diluents, or lubricating, surface active or dispersing agents. Molded tablets may be made by molding in a suitable machine a mixture of the powdered compound moistened with a liquid diluent. Dyes or pigments may be added to tablets for identification or to characterize different combinations of active compound doses.

Liquid formulations can include emulsions, solutions, syrups, elixirs and suspensions, which can be used in soft or hard capsules. Such formulations may include a pharmaceutically acceptable carrier, for example, water, ethanol, polyethylene glycol, cellulose, or an oil. The formulation may also include one or more emulsifying agents and/or suspending agents.

Compositions for oral administration may be formulated as immediate or modified release, including delayed or sustained release, optionally with enteric coating.

In another embodiment, a pharmaceutical composition comprises a therapeutically effective amount of a compound of Formula (I) or a pharmaceutically acceptable salt thereof, and a pharmaceutically acceptable carrier.

### Parenteral Administration

Compounds of the present invention may be administered directly into the blood stream, muscle, or internal organs by injection, e.g., by bolus injection or continuous infusion. Suitable means for parenteral administration include intravenous, intra-muscular, subcutaneous intraarterial, intraperitoneal, intrathecal, intracranial, and the like. Suitable devices for parenteral administration include injectors (including needle and needle-free injectors) and infusion methods. The formulations may be presented in unit-dose or multi-dose containers, for example sealed ampoules and vials.

Most parenteral formulations are aqueous solutions containing excipients, including salts, buffering, suspending, stabilizing and/or dispersing agents, antioxidants, bacteriostats, preservatives, and solutes which render the formulation isotonic with the blood of the intended recipient, and carbohydrates.

Parenteral formulations may also be prepared in a dehydrated form (e.g., by lyophilization) or as sterile non-aqueous solutions. These formulations can be used with a suitable vehicle, such as sterile water. Solubility-enhancing agents may also be used in preparation of parenteral solutions.

Compositions for parenteral administration may be formulated as immediate or modified release, including delayed or sustained release. Compounds may also be formulated as depot preparations. Such long acting formulations may be administered by implantation (for example subcutaneously or intramuscularly) or by intramuscular injection. Thus, for example, the compounds may be formulated with suitable polymeric or hydrophobic materials (for example as an emulsion in an acceptable oil) or ion exchange resins, or as sparingly soluble derivatives, for example, as a sparingly soluble salt.

### Topical Administration

Compounds of the present invention may be administered topically (for example to the skin, mucous membranes, ear, nose, or eye) or transdermally. Formulations for topical administration can include, but are not limited to, lotions, solutions, creams, gels, hydrogels, ointments, foams, implants, patches and the like. Carriers that are pharmaceutically acceptable for topical administration formulations can include water, alcohol, mineral oil, glycerin, polyethylene glycol and the like. Topical administration can also be performed by, for example, electroporation, iontophoresis, phonophoresis and the like.

Typically, the active ingredient for topical administration may comprise from 0.001% to 10% w/w (by weight) of the formulation. In certain embodiments, the active ingredient may comprise as much as 10% w/w; less than 5% w/w; from 2% w/w to 5% w/w; or from 0.1% to 1% w/w of the formulation.

Compositions for topical administration may be formulated as immediate or modified release, including delayed or sustained release.

### Rectal, Buccal, and Sublingual Administration

Suppositories for rectal administration of the compounds of the present invention can be prepared by mixing the active agent with a suitable non-irritating excipient such as cocoa butter, synthetic mono-, di-, or triglycerides, fatty acids, or polyethylene glycols which are solid at ordinary temperatures but liquid at the rectal temperature, and which will therefore melt in the rectum and release the drug.

For buccal or sublingual administration, the compositions may take the form of tablets, lozenges, pastilles, or gels formulated in conventional manner. Such compositions may comprise the active ingredient in a flavored basis such as sucrose and acacia or tragacanth.

### Administration by Inhalation

For administration by inhalation, compounds may be conveniently delivered from an insufflator, nebulizer pressurized packs or other convenient means of delivering an aerosol spray or powder. Pressurized packs may comprise a suitable propellant such as dichlorodifluoromethane, trichlorofluoromethane, dichlorotetrafluoroethane, carbon dioxide or other suitable gas. In the case of a pressurized aerosol, the dosage unit may be determined by providing a valve to deliver a metered amount. Alternatively, for administration by inhalation or insufflation, the compounds according to the disclosure may take the form of a dry powder composition, for example a powder mix of the compound and a suitable powder base such as lactose or starch. The powder composition may be presented in unit dosage form, in for example, capsules, cartridges, gelatin or blister packs from which the powder may be administered with the aid of an inhalator or insufflator.

Other carrier materials and modes of administration known in the pharmaceutical art may also be used. Pharmaceutical compositions of the invention may be prepared by any of the well-known techniques of pharmacy, such as effective formulation and administration procedures. Preferred unit dosage formulations are those containing an effective dose, as herein recited, or an appropriate fraction thereof, of the active ingredient. The precise amount of compound administered to a patient will be the responsibility of the attendant physician. The specific dose level for any particular patient will depend upon a variety of factors including the activity of the specific compound employed, the age, body weight, general health, sex, diets, time of administration, route of administration, rate of excretion, drug combination, the precise disorder being treated, and the severity of the indication or condition being treated. In addition, the route of administration may vary depending on the condition and its severity. The above considerations concerning effective formulations and administration procedures are well known in the art and are described in standard textbooks. Formulation of drugs is discussed in, for example, Hoover, John E., Remington's Pharmaceutical Sciences, Mack Publishing Co., Easton, Pa., 1975; Liberman, et al., Eds., Pharmaceutical Dosage Forms, Marcel Decker, New York, N.Y., 1980; and Kibbe, et al., Eds., Handbook of Pharmaceutical Excipients (3rd Ed.), American Pharmaceutical Association, Washington, 1999. **Methods of Treatment** (intended as compounds for use in the methods of treatment of the claimed diseases)

The methods of the invention can be used to treat any subject in need of treatment. Examples of subjects or patients include, but are not limited to, humans, monkeys, deer, camel, pets and companion animals, including, but not limited to, dogs, cats, horses, rabbits, and guinea pigs; livestock, including, but not limited to, cows, buffalo, bison, mules, goats, sheep and pigs. In one embodiment, the subject is a human.

The methods of the invention provide for the administration of a glutaminase inhibitor or a compound that inhibits glutathione production as disclosed above for use in the treatment of lung cancer wherein the NRF2/KEAP1 pathway in the cancer cells is deregulated.

In another embodiment, provided herein is glutaminase inhibitor or a compound that inhibits glutathione production as disclosed above for use as a medicament in the treatment of a lung cancer wherein the NRF2/KEAP1 pathway in the cancer cells is deregulated, including non-small cell lung cancer (NSCLC).

In some embodiments, the lung cancer has developed resistance to chemotherapeutic drugs and/or ionizing radiation.

### Combinations and Combination Therapy

The compounds of the present invention can be used, alone or in combination with other pharmaceutically active compounds, to treat conditions such as those disclosed hereinabove. The compound(s) of the present invention and other pharmaceutically active compound(s) can be administered simultaneously (either in the same dosage form or in separate dosage forms) or sequentially.

The glutaminase inhibitor or a compound that inhibits glutathione production can be used as a medicament in combination with one or more additional pharmaceutically active compounds. The glutaminase inhibitor or a compound that inhibits glutathione production and one or more additional pharmaceutically active compounds can be used for the manufacturing of a medicament. For example, the glutaminase inhibitor or a compound that inhibits glutathione production and one or more additional pharmaceutically active compounds can be used for the manufacturing of a medicament for the treatment of a disease or disorder according to the claims.

There is also provided a pharmaceutical composition comprising one or more compounds of the present invention, one or more additional pharmaceutically active compounds, and a pharmaceutically acceptable carrier.

The one or more additional pharmaceutically active compounds is chosen from anti-cancer drugs, anti-proliferative drugs, and antiinflammatory drugs. In certain embodiments, the anti-cancer agent is chosen from a platinum-based agent, a taxane-based agent, an immunotherapy, and a targeted therapy. In certain embodiments,the targeted therapy is an inhibitor of MEK kinase, HSP90, CDK4, or the mTOR pathway.

Glutaminase inhibitors, e.g., GLS-1 inhibitors, disclosed herein are also optionally used in combination with other therapeutic reagents that are selected for their therapeutic value for the condition to be treated. In general, the compounds described herein and, in embodiments where combination therapy is employed, other agents do not have to be administered in the same pharmaceutical composition and, because of different physical and chemical characteristics, are optionally administered by different routes. The initial administration is generally made according to established protocols and then, based upon the observed effects, the dosage, modes of administration and times of administration subsequently modified. In certain instances, it is appropriate to administer a glutaminase inhibitor compound, as disclosed herein, in combination with another therapeutic agent. By way of example only, the therapeutic effectiveness of a glutaminase inhibitor is enhanced by administration of another therapeutic agent (which also includes a therapeutic regimen) that also has therapeutic benefit. Regardless of the disease, disorder or condition being treated, the overall benefit experienced by the patient is either simply additive of the two therapeutic agents or the patient experiences an enhanced (i.e., synergistic) benefit. Alternatively, if a compound disclosed herein has a side effect, it may be appropriate to administer an agent to reduce the side effect; or the therapeutic effectiveness of a compound described herein may be enhanced by administration of an adjuvant.

Therapeutically effective dosages vary when the drugs are used in treatment combinations. Methods for experimentally determining therapeutically effective dosages of drugs and other agents for use in combination treatment regimens are documented methodologies. Combination treatment further includes periodic treatments that start and stop at various times to assist with the clinical management of the patient. In any case, the multiple therapeutic agents (one of which is a glutaminase inhibitor, e.g., a GLS-1 inhibitor, as disclosed herein) may be administered in any order, or simultaneously. If simultaneously, the multiple therapeutic agents are optionally provided in a single, unified form, or in multiple forms (by way of example only, either as a single pill or as two separate pills).

In some embodiments, one of the therapeutic agents is given in multiple doses, or both are given as multiple doses. If not simultaneous, the timing between the multiple doses optionally varies from more than zero weeks to less than twelve weeks.

In addition, the combination methods, compositions and formulations are not to be limited to the use of only two agents, the use of multiple therapeutic combinations are also envisioned. It is understood that the dosage regimen to treat, prevent, or ameliorate the condition(s) for which relief is sought, is optionally modified in accordance with a variety of factors. These factors include the disorder from which the subject suffers, as well as the age, weight, sex, diet, and medical condition of the subject. Thus, the dosage regimen actually employed varies widely, in some embodiments, and therefore deviates from the dosage regimens set forth herein.

The pharmaceutical agents which make up the combination therapy disclosed herein are optionally a combined dosage form or in separate dosage forms intended for substantially simultaneous administration. The pharmaceutical agents that make up the combination therapy are optionally also administered sequentially, with either agent being administered by a regimen calling for two-step administration. The two-step administration regimen optionally calls for sequential administration of the active agents or spaced-apart administration of the separate active agents. The time between the multiple administration steps ranges from a few minutes to several hours, depending upon the properties of each pharmaceutical agent, such as potency, solubility, bioavailability, plasma half-life and kinetic profile of the pharmaceutical agent.

In another embodiment, a glutaminase inhibitor, e.g., a GLS-1 inhibitor, is optionally used in combination with procedures that provide additional benefit to the patient. A glutaminase inhibitor and any additional therapies are optionally administered before, during, or after the occurrence of a disease or condition, and the timing of administering the composition containing a glutaminase inhibitor, e.g., a GLS-1 inhibitor, varies in some embodiments. Thus, for example, a glutaminase inhibitor is used as a prophylactic and is administered continuously to subjects with a propensity to develop conditions or diseases in order to prevent the occurrence of the disease or condition. A glutaminase inhibitor, e.g., a GLS-1 inhibitor, and compositions are optionally administered to a subject during or as soon as possible after the onset of the symptoms.

A glutaminase inhibitor, e.g., a GLS-1 inhibitor, disclosed herein can be used in combination with anti-cancer drugs, including the following classes alkylating agents, anti-metabolites, plant alkaloids and terpenoids, topoisomerase inhibitors, cytotoxic antibiotics, angiogenesis inhibitors and tyrosine kinase inhibitors.

For use in the treatment or attenuation of cancer and neoplastic diseases a glutaminase inhibitor may be optimally used together with one or more of the following non-limiting examples of anti-cancer agents, including: (1) alkylating agents, including but not limited to platinum-based agents such as cisplatin (PLATIN), carboplatin (PARAPLATIN), and oxaliplatin (ELOXATIN), as well as other alkylating agents such as streptozocin (ZANOSAR), busulfan (MYLERAN) and cyclophosphamide (ENDOXAN); (2) anti-metabolites, including mercaptopurine (PURINETHOL), thioguanine, pentostatin (NIPENT), cytosine arabinoside (ARA-C), gemcitabine (GEMZAR), fluorouracil (CARAC), leucovorin (FUSILEV) and methotrexate (RHEUMATREX); (3) plant alkaloids and terpenoids, including vincristine (ONCOVIN), vinblastine and paclitaxel (TAXOL); (4) topoisomerase inhibitors, including irinotecan (CAMPTOSAR), topotecan (HYCAMTIN) and etoposide (EPOSIN); (5) cytotoxic antibiotics, including actinomycin D (COSMEGEN), doxorubicin (ADRIAMYCIN), bleomycin (BLENOXANE) and mitomycin (MITOSOL); (6) angiogenesis inhibitors, including sunitinib (SUTENT) and bevacizumab (AVASTIN); (7) tyrosine kinase inhibitors, including imatinib (GLEEVEC), erlotinib (TARCEVA), lapatininb (TYKERB) and axitinib (INLYTA) ; 8) Hsp90 inhibitors, including Ganetesipib; 9) MEK inhibitors, including Trametinib and Salumetinib; 10) CDK4/6 inhibitors, including Palbociclib; 11) mTor inhibitors, including Sirolimus and Everolimus; 12) mTORC1/2 inhibitors, including AZD-8055; and 13) Immune Checkpoint Regulators including Ipilimumab and Nivolumab

Where a subject is suffering from or at risk of suffering from an inflammatory condition, a glutaminase inhibitor, e.g., a GLS-1 inhibitor, compound disclosed herein is optionally used together with one or more agents or methods for treating an inflammatory condition in any combination. Therapeutic agents/treatments for treating an autoimmune and/or inflammatory condition include, any of the following examples: (1) corticosteroids, including cortisone, dexamethasone, and methylprednisolone; (2) nonsteroidal anti-inflammatory drugs (NSAIDs), including ibuprofen, naproxen, acetaminophen, aspirin, fenoprofen (NALFON), flurbiprofen (ANSAID), ketoprofen, oxaprozin (DAYPRO), diclofenac sodium (VOLTAREN), diclofenac potassium (CATAFLAM), etodolac (LODINE), indomethacin (INDOCIN), ketorolac (TORADOL), sulindac (CLINORIL), tolmetin (TOLECTIN), meclofenamate (MECLOMEN), mefenamic acid (PONSTEL), nabumetone (RELAFEN) and piroxicam (FELDENE); (3) immunosuppressants, including methotrexate (RHEUMATREX), leflunomide (ARAVA), azathioprine (IMURAN), cyclosporine (NEORAL, SANDIMMUNE), tacrolimus and cyclophosphamide (CYTOXAN); (4) CD20 blockers, including rituximab (RITUXAN): (5) Tumor Necrosis Factor (TNF) blockers, including etanercept (ENBREL), infliximab (REMICADE) and adalimumab (HUMIRA); (6) interleukin-1 receptor antagonists, including anakinra (KINERET); (7) interleukin-6 inhibitors, including tocilizumab (ACTEMRA); (8) interleukin-17 inhibitors, including AIN457; (9) Janus kinase inhibitors, including tasocitinib; and (10) syk inhibitors, including fostamatinib.

### General Synthetic Methods for Preparing Compounds

Compounds useful in the methods of the present invention can be prepared using methods that are known to one of skill in the art. Starting materials used to prepare compounds of the present invention are commercially available or can be prepared using routine methods known in the art.

### Biological Assays

The following are examples of biological assays useful with the methods of the invention. The assays provided herein are not limiting and other assays now known, or later discovered, by one of skill in the art can be used for the same purpose as the assays provided below.

Compounds disclosed herein are active as GLS-1 inhibitors. Compounds disclosed in Table 1 were synthesized and tested, and had IC50s of less than 100nM.

Compounds disclosed herein are also active in inhibiting A549 cancer cell proliferation. Compounds disclosed in Table 1 were synthesized and tested, and had IC50s of less than 100nM. These compounds are expected to be active against other cancers disclosed herein, such as those wherein NRF2/KEAP1 pathway in the cancer cells is deregulated, NRF2 signaling in the cancer cells is hyperactive, the nucleic acid of the cancer cells is comprises a loss-of-function mutation in KEAP1, the nucleic acid of said subject comprises a gain-of-function mutation in NRF2, the cancer cells have increased intracellular concentration of glutathione.

### Glycerol Lysis Buffer Recipe

| | Final Concentration |
|---|---|
| 2ml 1M Tris-HCl pH 7.4 | 20mM |
| 3ml 5M NaCl | 150mM |
| 15ml 100% glycerol | 15% |
| 1ml 100% Triton-X 100 | 1% |
| 79 ml Distilled Water | (100ml total) |

To each 1ml add before use: 100ul 10% SDS, 5µl 200mM PMSF, 10ul HALT Protease, 1ul of Benzonase

### Cell Viability Assay

Cell culture media: All cells were grown in RPMI-1640 (Gibco11875-119) with 2mM Glutamine + 10% FBS (Gibco16000-044) unless otherwise noted.

Cells were plated in black, 96-well Corning Costar 3603 at densities to allow for Log phase growth throughout the experiment (see table below) on Day 0. Cells were allowed to settle and attach overnight. Cells plated in triplicate for each concentration and control examined. On Day 1, media was removed from cells and replaced with RPMI-1640 + 10% Dialyzed FBS (Gibco26400-044) with indicated concentration of IACS compound (starting concentration 1µM, diluted 1:3 to a final of 0.004µM), 0.01%DMSO or 1µM Staurosporine as indicated. Cells were incubated with compound or relevant controls for 72 hours. On Day 4, 72 hours after treatment, cells were analyzed by Cell Titer Glo (PromegaG7573) per manufacturer's instructions. Plates were read in luminescence mode on a PheraStarFS plate reader. Data was collected, replicates were averaged, standard deviations calculated and analyzed through nonlinear regression analysis with four (4) parameters to generate IC50s for each cell line tested.

| **Cell Line** | **Cells Plated Per Well (96-well)** |
|---|---|
| A549 | 4,000 |
| NCI-H2228 | 6,000 |
| H727 | 15,000 |
| H1650 | 5,000 |
| NCI-H2122 | 8,000 |
| H292 | 5,000 |
| HCC15 | 5,000 |
| H441 | 10,000 |
| H1395 | 15,000 |
| NCI-H1568 | 5,000 |
| NCI-H520 | 5,000 |
| H460 | 4,000 |
| H1792 | 5,000 |
| H1944 | 10,000 |
| H2023 | 4,000 |
| H2030 | 5,000 |
| H2170 | 4,000 |
| H1299 | 5,000 |
| HOP92 | 5,000 |
| H209 | 5,000 |
| HCC-78 | 5,000 |
| Colo699 | 5,000 |
| H115 | 5,000 |

Analysis of Glutathione Levels: Cells were plated in 96-well Corning Costar 3603 at twice the density in the table shown above on Day 0. Cells were allowed to settle and attach overnight. Cells plated in triplicate for each concentration and control examined. On Day 1, media was removed from cells and replaced with RPMI-1640 + 10% Dialyzed FBS (Gibco26400-044) with indicated concentration of IACS-011393 (starting concentration 1uM, diluted 1:3 to a final of 0.004uM), or 0.01%DMSO as indicated. Cells were incubated with compound or relevant controls for 24 hours. On Day 2, 24 hours after treatment, cells were analyzed by GSH-Glo (PromegaV6912) per manufacturer's instructions. Plates were read in luminescence mode on a PheraStarFS plate reader. Duplicate wells were plated and then stained with crystal violet in order to correct for differences in cell growth as described below.

### GLS-1 Enzymatic Activity Assay

The following is a non-limiting example of an assay that may be used to evaluate the biological efficacy of compounds useful in the methods of the invention or to determine if a compound is a selective inhibitor of GLS-1.

The inhibition of purified recombinant human GAC by varying concentrations of inhibitors may be assessed via a dual-coupled enzymatic assay. The glutamate produced by the glutaminase reaction is used by glutamate oxidase to produce α-ketoglutarate, ammonia, and hydrogen peroxide. This hydrogen peroxide is subsequently used by horseradish peroxidase to produce resorufin in the presence of Amplex UltraRed. The assay buffer consisted of 50mM Hepes (pH 7.4), 0.25mM EDTA and 0.1mM Triton X-100. GAC was incubated with potassium phosphate (10 minutes at room temperature) prior to incubation with inhibitor (10 minutes at room temperature). The final reaction conditions were as follows: 2nM GAC, 50mM potassium phosphate, 100mU/mL glutamate oxidase (Sigma), 1mM glutamine (Sigma), 100mU/mL horseradish peroxidase (Sigma), 75µM Amplex UltraRed (Life Technologies), and 1% (v/v) DMSO. The production of resorufin was monitored on a Perkin Elmer Envision plate reader (excitation 530nm, emission 590nm) either in a kinetics or endpoint mode (at 20 minutes). IC₅₀ values were calculated using a four-parameter logistic curve fit.

### Proliferation Assay

A549 cells were routinely maintained in RPMI 1640 media (Gibco catalog number 11875-093) supplemented with 10% dialyzed fetal bovine serum using a humidified incubator (37oC, 5% CO₂ and ambient O₂). In preparation for the viability assay, cells were inoculated into 384-well black CulturPlates (Perkin Elmer) at a density of 1000 cells/well in a volume of 40uL. Following a 24-hour incubation at 37 °C, 5% CO2 and ambient O₂, cells were treated with compound (10uL) in a final DMSO concentration of 0.5% (v/v). The microplates were then incubated for 72 hours (37 °C, 5% CO₂ and ambient O₂). Cell Titer Fluor (Promega) was subsequently added (10uL of 6x reagent) and mixed for 15 minutes at room temperature. The plates were then incubated for 30 minutes (37 °C, 5% CO₂ and ambient O₂) and fluorescence was subsequently read on the Perkin Elmer Envision plate reader. IC₅₀ values were calculated using a four-parameter logistic curve fit.

### Target Engagement Assay

A549 cells were plated at 400,000 cells per well in a 6-well dish. H727 cells were plated at 800,000 cells per well in a 6 well dish. 24h after plating, media was removed and replaced with media containing 1µM IACS-011393 or 0.01%DMSO (control). A 0h time point was immediately collected by removing 250µl of media from each well into a 1.7mL Eppendorf tube. These samples were placed at -20 for storage. Subsequent collections for each time point were made in the same fashion. All samples are stored at -20 until the analysis step. At 24h, an additional 250µl of media was collected from each sample. Media was frozen after collection. At t=0 and t=24 cell lysates were collected through lysis with glycerol lysis buffer (see below) by adding 200ul of lysis buffer to each well, incubating at 37 degrees for 5 min, and collecting all lysate into a microfuge tube. At each time point, one well for each condition was fixed and stained with crystal violet to normalize for cell number (see protocol below).All crystal violet samples were solubilized after the final timepoint was collected and used to normalize the collected data. Collected samples were analyzed for glutamine and glutamate content using the YSI2950 Bio-analyzer equipped with membranes from measure the relevant chemistries.

### Crystal Violet Staining Protocol

500mg crystal violet powder was combined with 35ml 100% EtOH in sterile 50ml tube until it dissolved. The mixture was transferred to a 1L beaker, the tube washed out with ddH₂O, and ddH₂O was added to the beaker to 500ml final volume. The mixture was filter sterilized using a 0.45µm filter. Media was removed and discarded (including wells without cells for background). 50µL crystal violet stain + 10% EtOH was added to the wells and stained for 10min. The stain was removed from the wells and discarded into a waste container. Water was run in a large beaker or ice bucket and the plate was repeatedly rinsed in the water. The remaining liquid was shaken out into the sink. The plate was left upside down, uncovered for at least 5 hours to dry. Once completely dry, it was solubilized in 10% acetic acid by adding 100µL to all stained wells. After 5 minutes, all wells were pipet mixed and after another 5 minutes read on Pherastar at OD 590nm.

### DNA Damage Staining and Quantification

Cells were seeded in 96-well Corning Costar 3603 plates and treated with 0.01% DMSO or 1µM IACS-011393 for 48 hours. Cells were then fixed using 4% Paraformaldehyde and permeabilized using 0.5% TritonX100. Fixed cells were stained with Anti-γH2AX, rabbit (Bethyl IHC-00059)(1:500) for 2h at 37C. The secondary antibody used was Alexa Fluor 546 donkey anti-rabbit (Invitrogen A10040)(1: 150) simultaneously with Hoechst 33342, Trihydrochloride, Trihydrate (Invitrogen, H3570)(1: 1000) for 1h at 37C.DNA damage was measured as a γH2AX signal per nucleus using PerkinElmer High Content Screening System Operetta with the Harmony software. First, nuclei were selected using Hoechst staining. Then, single cells were gated based on nucleus size and shape. Apoptotic and mitotic cells were excluded based on maximum Hoechst intensity in the nucleus. DNA damage foci were identified within the gated nuclei using the "find spots in Alexa 546 channel" analysis block. Finally, the total intensity of Alexa 546 within all "spots" in the nucleus, or the total number of spots in the nucleus, averaged per cell, was calculated.

### Metabolomics Sample Preparation

A549 cells were plated at 4.5×10⁶ cells per 10cm dish 24h prior to treatment. Cells were treated on day zero (d.0) at time zero (t.0) with 0.01% DMSO, 1µM, 100nM, or 10nM of IACS-011393 for 24h. On day 1 (d.1), 2 hours prior to harvest, samples were washed with 5mL media containing 10% dialyzed FBS.10mL of media was added to plates and plates were returned to the incubator for 2h. Media was then aspirated from plates and 4mL of 80% chilled MeOH (-80°C) was immediately added. Plates were incubated at -80°C for 15 minutes. Cells were then scraped with cell scraper to release cells while keeping plates on dry ice. The MeOH/cell lysate mixture was collected and transferred to conical tubes on dry ice. Tubes were centrifuged at full speed in 4°C chilled centrifuge for 5 minutes. Supernatant was transferred to another set of conical tubes on dry ice by decanting. The remaining pellet was re-suspended in 500µL of cold MeOH and the mixture was moved to a microcentrifuge tube. Microfuge tubes were spun at full speed for 5 minutes in 4°C chilled microcentrifuge. Supernatant was transferred to conical tube with previously collected supernatant. This step was repeated 2 times. 1mL of collected supernatant was transferred to pre-labeled sample/submission tubes and submit for drying by speedvac. Samples were then submitted to the Beth Israel Deaconess Medical Center Mass Spectrometry core facility for analysis.

### Metabolomics Sample Analysis

Differentially abundance analysis of metabolite levels was carried out using a moderated t-test function from Bioconductor's limma package (Smyth 2005). A normalization factor was applied to the metabolite peak data to account of variation in cell counts. The normalization factor was calculated by first defining a vector, Nmed, to be the median peak area for each metabolite across a set of samples. Next, for a sample i, a vector of fold-changes, FCi, is computed by dividing the metabolite peak areas by Nmed. The scaling factor for sample i was set as the median value of FCi. Significant changes in metabolite levels were defined an abundance change of a least 1.5 fold increase / decrease and t-test p-value <= 0.05. The symbols were assigned according to the p-values with the following cutoffs: + = p > 0.005 and p <= 0.05, ^{∗} = p > 0.00005 and p <= 0.005, ^{∗∗} = p <= 0.00005.

### Metabolic Flux and LC/MS Sample Analysis

A549 cells were plated at 4.5×10⁵ cells per 6cm dish 24h prior to treatment. On the day of the experiment, cells were treated with 0.01% DMSO, or 1µM of IACS-011393 for 6h in the presence or absence of fully labeled ¹³C-Glutamine (Cambridge Isotope Laboratories, Inc., CLM01822). Labeled glutamine was added in regular media containing no unlabeled glutamine, such that cells could only metabolize the labeled form. After treatment, media was then aspirated from plates, plates were washed 2x in cold PBS, and 500µL of 80% chilled MeOH (-80°C) was immediately added. Plates were incubated at -80°C for 15 minutes. Cells were then scraped with cell scraper to release cells while keeping plates on dry ice. The MeOH/cell lysate mixture was collected and transferred to conical tubes on dry ice. Tubes were centrifuged at full speed in 4°C chilled centrifuge for 5 minutes. Supernatant was transferred to another set of conical tubes on dry ice by decanting. The remaining pellet was re-suspended in 500uL of cold MeOH and the mixture was moved to a microcentrifuge tube. Microfuge tubes were spun at full speed for 5 minutes in 4°C chilled microcentrifuge. Supernatant was transferred to conical tube with previously collected supernatant. And this step was repeated 2 times. Collected supernatant was dried by speedvac. Dry samples were reconstituted with acetonitrile/water (1:1) and analyzed in an Agilent 1209 HPLC system coupled to an Agilent 6550 quadrupole time-of-flight (Q-TOF) mass spectrometer. Samples (10 µL) were injected into a Waters XBridge Amide column for HILIC/MS analysis. The mobile phases A, 20 mM ammonium acetate, 20 mM acetic acid in 95% water/acetonitrile and B, 100% acetonitrile were used with a linear gradient elution from 85% B to 100 % A for a total sample run time of 30 min. The raw data was processed using Agilent Mass Hunter Qualitative Analysis. Isotopologues of putative metabolites incorporating carbons derived from [U-¹³C]-glutamine were identified based on their accurate masses and retention times which were further confirmed with those of authentic standards and public database compound libraries.

### Nrf2 Score

Single sample gene set enrichment (ssGSEA) was used to score NRF2 activity in lung cancer cell lines with expression data published as part of the Cancer Cell Line Encyclopedia (CCLE). ssGSEA assigns an enrichment based on the distribution of a gene set relative all other genes within a single sample (Barbie et al, 2009). The ssGSEA enrichment scores were median centered and normalized by the standard deviation of the enrichment scores from all lung cell lines in the CCLE panel. The NRF2 signature gene set was based on a known set of NRF2 target genes: ABCC1, ABCC2, G6PD, GCLC, GCLM, GRK6, GSR, GSTM4, HMOX1, ME1, MGST1, NQO1, PRDX1, TXN and TXNRD1.

### Gene Expression Analysis

Affymetrix U133 Plus2.0 microarrays were performed for each condition in triplicates. Robust multi-array average (RMA) method was used with default options (with background correction, quantile normalization, and log transformation) to normalize raw data from batches using R/Bioconductor's affy package (Irizarry et al 2003). Differentially expression analysis was carried out using a moderated t-test function from Bioconductor's limma package (Smyth 2005). A gene is called as differentially expressed if FDR corrected p-value is less than 0.05.

### Analysis of Reactive Oxygen Species Production

One day prior to the experiment, A549 cells were seeded in 6 well dishes (250,000 cells per well). The day of the experiment, cells were treated with 0.01% DMSO or 1µM IACS-011393 for 24 hours. The following day, an allocated well was treated with hydrogen peroxide for 20-30 minutes as a positive control for ROS generation. All wells were then stained with CM-H2DCFDA (5 µM, Life Technologies) for 30 minutes at 37°C, washed with IX PBS, harvested by trypsinization, and re-suspended in 400 µl phenol-free RPMI medium. Cells were strained through a 40 µM cell strainer and analyzed using a flow cytometer (LSR Fortessa). Values displayed are fold changes compared to DMSO for mean fluorescence intensity of entire population.

### EXAMPLES

Several embodiments of the present invention are provided in the following examples. The following examples are presented only by way of illustration and to assist one of ordinary skill in using the invention. The examples are not intended in any way to otherwise limit the scope of the invention.

### Example 1. NSCLC cell lines are differentially sensitive to GLS inhibition.

To investigate the role of GLS in regulating NSCLC cell proliferation we first characterized a chemical probe designed to specifically inhibit GLS-1. We have confirmed that the reference compound (IACS-011393) is a potent inhibitor of GLS-1 (IC₅₀ 7.5nM) in a dual-coupled in vitro enzymatic assay (Figure 1A). The inhibition of GAC by varying concentrations of IACS-011393 was assessed using a dual-coupled enzymatic assay (pH 7.4) containing 2nM GAC, 50mM potassium phosphate, 100mU/mL glutamate oxidase, 1mM glutamine, 100mU/mL HRP and 75µM Amplex Ultra Red. The production of resorufin was monitored on a Perkin Elmer Envision plate reader (excitation 530nm, emission 590nm) for 20 min.

In cells, the chemical probe inhibits the conversion of glutamine to glutamate and reduces glutamine-dependent oxygen consumption, both measures of GLS-dependent activity (Fig. 1B, 1C). Figure 1B shows that target engagement can be measured after treatment with IACS-011393. As a measure of compound activity on GLS in cells, the conversion of glutamine to glutamate was measured in extracellular media and cell lysates in samples treated with DMSO (control) or IACS-011393. Treatment of cells for 24 hours with IACS-011393 prevented the conversion of glutamine to glutamate (as measured by the ratio of glutamate to glutamine (GLU:GLN)). This inhibition was evident by a decrease in the GLU:GLN ratio outside the cell and in cell lysates.

Figure 1C shows that target A549 cells utilize glutamine anaplerosis to drive respiration. A549 cells were plated and then starved of nutrients for 60 minutes. After starvation, cells were pre-loaded with the indicated concentrations of IACS-011393 to inhibit GLS. Next, 2mM glutamine was added to the wells to trigger oxygen consumption, and oxygen consumption rate (OCR) was quantified by Seahorse analysis after 20 minutes. IACS-011393 shows a dose-dependent inhibition of glutamine-dependent oxygen consumption, suggesting that A549 cells utilize glutamine anaplerosis as a metabolite source for the TCA cycle. This process is blocked by the glutaminase inhibitor ("GLSi").

We next sought to determine the potential therapeutic benefit of GLS inhibition in NSCLC. Through a cell screening campaign, we identified a subpopulation of NSCLC cell lines that are hypersensitive to GLS inhibition (IC₅₀ 2D viability = 5nM in "responders" ; >10µM in "non-responders") (Fig. 1D). Figure 1D shows differential sensitivity to IACS-011393 is seen in various NSCLC lines. A549, NCI-H2122, H1650 and H1395 NSCLC lines were treated with non-claimed compound IACS-011393 as in (A) above and 72 hour viability was analyzed. While A549 and H2122 show nM sensitivity to GLSi, H1395 and H1650 cells are resistant to treatment with GLSi. This suggests a differential sensitivity with possible genetic variance between these lines driving sensitivity. Interestingly, nearly all lines show a depletion of intracellular glutamate levels indicative of glutaminase inhibition. This suggests that a subset of NSCLC cell lines have a specific dependence on glutamine metabolism for survival.

### Example 2. GLS inhibition alters redox balance in responder cell lines.

To interrogate the role of Glutaminase in the regulation of tumor metabolism, targeted liquid chromatography-tandem mass spectrometry (LC-MS/MS) metabolomic studies were performed to comprehensively characterize metabolic alterations following GLS inhibition (Fig. 2). A549 cells treated with 1 µM of non claimed compound IACS-011393 for 24 hours were subjected to global metabolic profiling after isolation of lysates by methanol extraction. Changes in metabolite levels reflected an accumulation of glutamine in cells after GLSi accompanied by a decrease in TCA cycle activity; likely due to decreased glutamine anaplerosis.

Additionally, decreases in free nucleotide pools were seen. These decreased metabolic activities were accompanied by an increase in pentose phosphate pathway activity, presumably to attempt to maintain redox balance in the absence of glutathione derived from the GLS-dependent breakdown of glutamine to glutamate, and activation of a ribose salvage pathway in response to IACS-011393 treatment, suggesting that cells are attempting to maintain nucleotide and thiol pools in response to decreased glutaminolysis. It should be noted that these metabolic changes are muted or absent in non-responder lines treated with IACS-011393 as shown in plots for metabolite levels in H727 cells (a non-responder cell line). Also of note, in non-responder cell lines, such as H727 cells, consumption of alanine and aspartate, presumably to re-generate intracellular pools of glutamate, are up-regulated in response to GLSi. This suggests that non-responder cells are less dependent upon glutaminase-derived pools of glutamate for survival. Significant changes in metabolite levels were defined an abundance change of a least 1.5 fold increase / decrease and t-test p-value <= 0.05. The symbols were assigned according to the p-values with the following cutoffs: + = p > 0.005 and p <= 0.05; ^{∗} = p > 0.00005 and p <= 0.005; ^{∗∗} = p <= 0.00005. Abbreviations - aminoimidazole carboxamide ribonucleotide(AICAR), inosine monophosphate (IMP) ribose 5-phosphate (R5P), sedoheptulose-1-7-phosphate (S7P), sedoheptulose-1,7-bisphosphate (SBP), glyceraldehdye-3-phosphate (G3P), dihydroxy-acetone-phosphate (DHAP), erythrose-4-phosphate (E4P).

Analysis of stable isotope labeled carbon flux from glutamine metabolism reveals a contribution to glutamate and glutathione synthesis in A549 cells (Fig. 2B). A549 cells were grown in the presence of DMSO or IACS-011393 in media containing fully labeled ¹³C-glutamine as the sole glutamine source. After 6 hours, cell lysates were extracted and analyzed by LC/MS to examine the incorporation of stably labeled carbons into metabolites within cells. As shown in the left panel, fully labeled glutamate is present in cells that have been treated with DMSO. However, in cells treated with IACS-011393, there are no labeled carbons in the glutamate pools remaining in the cell (m+5, white bars). Additionally, DMSO treated cells contain labeled carbons in their pools of glutathione, while cells treated with IACS-011393 do not. These results suggest that inhibition of glutaminase prevents the conversion of glutamine to glutamate and subsequently the inclusion of glutamate into glutathione pools, thus altering redox balance within responder cells.

When GLS is inhibited in cells, labeled carbons are absent from glutamate and glutathione pools, suggesting that GLSi prevents glutamine-derived glutathione synthesis (Fig. 2C). Fig. 2C shows a schematic representation of the incorporation of carbons from fully labelled 13C-Glutamine into glutathione in the absence or presence of GLSi - A schematic representation of the results presented in Fig. 2B. Dark circles represent labelled carbon atoms in cells with functional glutaminase (left) or cells treated with a glutaminase inhibitor (right).

This analysis revealed that GLS inhibition in "responder" cell lines induced significant metabolic changes involving multiple pathways, the most significant of which are intermediates in the TCA cycle and altered redox balance. Consistent with the role of glutamine in regulating intracellular redox balance, GLS inhibition decreased the effective concentration of glutathione, and induced activity of the pentose phosphate and ribose salvage pathways. Interestingly, changes in these metabolic pathways were muted in "non-responder" NSCLC lines, confirming a differential addiction to glutamine.

### Example 3. NRF2/KEAP1 pathway modulation confers sensitivity to GLS inhibition.

We sought molecular insight into the differential response to GLS inhibition. Given the observed metabolic shift towards pathways involved in redox balance, we focused our attention on genetic modifiers of antioxidant defense. Genetic analysis showed that many GLSi responder cell lines harbored somatic alterations within the NRF2/KEAP1 pathway. NRF2 is a transcription factor that binds the Antioxidant Response Element (ARE) and initiates transcription of a number of genes involved in regulation of redox balance and cellular detoxification. KEAP1 is a negative regulator of NRF2, maintaining activity through induced proteosomal degradation.

A panel of NSCLC lines was tested for response to non-claimed compound IACS-011393 as described in Example 2 above. Cell lines with loss-of-function mutations in Keap1 or gain-of-function mutations in Nrf2 were sensitive to GLS inhibition with IACS-011393 while most cells lacking activating mutations in this pathway were not sensitive (Fig. 3A). Mutations in responder lines fall within the domains mediating the interaction between Keap1 and Nrf2. These mutations cause hyperactive Nrf2 signaling and an increased tolerance to redox stress within cells. Average IC₅₀ values are listed (n=3 to 10 for each individual line). Nrf2 score is a measure of the relative expression of Nrf2 target genes.

In NSCLC lines that respond to GLSi, this pathway is hyperactivated through loss of function mutations in KEAP1 or gain of function mutations in NRF2 which limit or prevent the interaction of these two proteins (Fig. 3B). The mutations in responder cell lines fall within important functional domains of Keap1 and Nrf2 as shown in a schematic representation of example mutations in Keap1 and Nrf2 in cell lines tested in Fig. 3A. Most mutations fall within important functional domains that regulate Keap1 activity, or Keap1-Nrf2 binding. The exception in the cell lines tested is the D77N mutation in NCI-H1568 cells which falls outside of one of two Keap1-binding domains in Nrf2. These data suggest that the loss of Keap1 regulation of Nrf2 results in hypersensitivity to GLSi.

Target engagement is seen in both responder and non-responder cell lines (Fig. 3C). Treatment of cells with IACS-011393 results in decreased conversion of glutamine to glutamate as monitored by the intracellular ratio of GLU:GLN in both responder and non-responder cell lines. Representative data is shown in Figure 3C for A549 cells (a responder line) and H727 cells (a non-responder line) suggesting that responder lines are hyper-dependent on GLS-mediated glutaminolysis, while non-responder lines are less dependent on this process.

Cells with hyperactive NRF2 signaling appear to be especially sensitive to GLSi due, in part, to the fact that NRF2 drives the expression of Glutamate-cysteine ligase (GCLC), the rate limiting enzyme that catalyzes glutathione synthesis from intracellular pools of glutamate and cysteine. We hypothesize that NRF2/KEAP1 mutant cells have evolved a dependence on glutathione to regulate redox balance. Thus, under conditions in which GLS and glutathione synthesis are inhibited, cells are unable to produce sufficient free radical scavengers to maintain redox balance within the cell. The up-regulation of the pentose phosphate and ribose salvage pathways observed through metabolic profiling suggest a futile compensatory effort to restore redox balance. Taken together, these data suggest that NRF2/KEAP1 pathway modulation sensitizes NSCLC cell lines to GLS inhibition.

### Example 4. GLS inhibition induces ROS and DNA damage in NRF2/KEAP1 mutant NSCLC cells.

The differential sensitivity of NRF2/KEAP1 mutant NSCLC cell lines suggested a role of glutathione in regulating redox balance. Consistent with this hypothesis, GLS inhibition induced a significant decrease in intracellular glutathione and a concurrent increase in reactive oxygen species (Fig. 4A, 4B). Figure 4A shows that glutathione levels are decreased after treatment with non-claimed compound IACS-011393 in responder cell lines. As Nrf2 drives the expression of glutamyl-cysteine ligase (GCLC) which synthesizes glutathione (GSH) from glutamate(GLU), glycine and cysteine, GSH levels were analyzed in cell lines after treatment with IACS-011393. GSH levels were significantly decreased in A549, H460 and H2122 cells after 24 hours of treatment (1µM). A representative non-responder line, H727, does not show a significant decrease in GSH levels after GLSi, suggesting that these cells have alternate mechanisms for maintaining redox balance. This supports the idea that Keap1/Nrf2 mutant cells are especially dependent on glutaminolysis for maintenance of redox balance.

Figure 4B shows that the loss of glutathione after treatment with a glutaminase inhibitor leads to an accumulation of intracellular reactive oxygen species (ROS). Cells were analyzed for accumulation of intracellular ROS after 24h treatment with IACS-011393 by staining with CM-H₂DCFDA (an indicator of general oxidative stress) and analysis by flow cytometry. For each condition, calculation of mean fluorescence intensity allows for the comparison of ROS accumulation between control and treated samples. Treatment of responder cells with IACS-011393 (1µM) for 24 hours induces an accumulation of ROS that correlates with the depletion of the GSH pool from cells. H727 cells show no such increase in ROS accumulation possibly due to their ability to maintain glutathione pools through alternative sources of glutamate generation.

We observed an accumulation of γH2AX foci, a marker of DNA damage, and the likely cause of anti-proliferative effects observed upon GLS inhibition (Fig. 4C). Figure 4C shows accumulation of DNA damage after non-claimed compound IACS-011393 treatment can be rescued by application of cell-permeable glutathione. A549 cells treated with either DMSO or IACS-011393 for 48 hours were stained with Hoechst's Stain (nuclei) and an antibody to γH2AX (DNA damage puncta) and then analyzed by high-content imaging. γH2AX foci were counted and quantified per nuclei. Quantification (representative images shown) reveals that IACS-011393 treatment induces DNA damage in A549 cells. Exogenous application of 4mM cell-permeable glutathione reduces the accumulation of DNA damage, presumably by scavenging ROS that has accumulated after IACS-011393 treatment.

GLSi-induced DNA damage and inhibition of cell proliferation were rescued upon treatment with exogenous glutathione, further strengthening the genetic association between GLS and NRF2 transcriptional activity. Figure 4D shows the results of A549 and H2122 cells that were treated with IACS-011393 at the indicated doses in a 72h growth assay. At the same time, a subset of the IACS-011393 treated cells received applications of cell-permeable GSH (once at the time of IACS-011393 treatment, once 24h post-treatment and once 48h post-treatment). Application of exogenous GSH rescued a significant portion of cell growth that was inhibited after IACS-011393 treatment. This data supports the idea that Keap1/Nrf2 mutant cell lines are especially sensitive to GLSi due to a heavy dependence on Nrf2-driven glutathione pools that allow these cells to grow under conditions of extreme oxidative stress.

Of note, neither glutathione depletion, ROS induction, nor an accumulation of DNA damage was observed in H727, a non-responder cell line. These data suggest that GLS inhibition in NRF2/KEAP1 mutant NSCLC cell lines inhibits cell growth by altering redox balance. The observed reduction in glutathione and increase in ROS was observed upon treatment with multiple, structurally distinct series of GLS inhibitors, such as non-claimed compound IACS-011393, non-claimed compound BPTES, non-claimed compound CB-839 and compound 13.

### Example 5. Acquired resistance to GLSi involves adaptive mechanisms that supply alternative sources of glutamate to maintain redox balance.

To further define the contribution of glutathione as rate limiting in NRF2/KEAP1 mutant NSCLC, we challenged cells with an IC90 dose (100nM) of non-claimed compound IACS-11393 for 8 weeks until resistant clones emerged. Rechallenge of either the bulk population (Fig. 5A) or selected single clones (Fig. 5B) confirmed a >3 log shift in IC50 values. To determine the mechanisms of adaptive resistance to GLSi treatment, we performed comprehensive metabolomic (Fig. 5C) and transriptomic analyses (Fig. 5D). Consistent with the defined role of GLS in maintaining redox balance, resistant clones were confirmed to (i) produce alternative sources of glutamate; (ii) activate alternative pathways to generate reducing power; (iii) and upregulate pathways that contribute intermediates to the TCA cycle (Fig. 5D).

Fig. 5A shows that cells treated chronically with GLSi acquire resistance to non-claimed compound IACS-011393. Bulk populations of A549 cells were carried in media containing increasing concentrations of GLSi over the course of two months up to a high concentration of 100nM. Cells became resistant to compound (A549-CT, left panel) compared to parental control cells (A549). This bulk population then underwent clonal selection in the presence of 100nM IACS-011393, and multiple drug resistant clones were identified with altered response to GLSi (right panel, individual clones compared to A549 parental line).

Fig. 5B shows that clones resistant to GLSi display activation of alternative pathways to maintain redox balance. Clones were subjected to baseline metabolomics analysis and compared to the parental A549 cell line. Log2-fold changes are shown for metabolites that were significantly up or down-regulated in comparison to baseline levels in the parental strain. These data indicate that (a) that clones have developed alternative sources of glutamate; (b) cells have activated alternative pathways to generate reducing power; (c) pathways that may contribute intermediates to the TCA cycle independent of glutaminolysis have been up regulated.

Fig. 5C shows that clones resistant to GLSi display altered transcriptional profiles to produce alternative sources of glutamate and reducing power. The same clones analyzed in Fig. 5B were subjected to RNA profiling and compared to A549 parental cells. Transcriptional changes (Log2 fold differences are displayed) across clones were consistent and showed a distinct up-regulation of enzymes and proteins involved in producing glutamate from sources other than glutaminase.

Fig. 5D provides a global overview of acquired adaptation to glutaminase inhibition. A global overview of metabolic and transcriptomic changes observed in cells resistant to chronic GLSi shows that A549 cells are able to initiate multiple pathways that provide reducing power to cells that are unable to convert glutamine to glutamate and glutathione through the activity of glutaminase. The main themes are alternative sources of glutamate, which may be used for glutathione synthesis and anaplerosis, along with an up-regulation of pathways involved in generating reducing intermediate (e.g., NADPH from malic enzyme). Bolded text represents metabolites or transcripts that were up relative to parental cells, while italicized text represents metabolites or transcripts that were down relative to parental cells. Abbreviations: S-adenosyl-L-methionine (SAM), S-adenosyl-L-homocysteine (SAH), homocysteine (hcys) , S-methyl-5-thioadenosine (MTA) aminoimidazole carboxamide ribonucleotide(AICAR), inosine monophosphate (IMP), ribose 5-phosphate (R5P), sedoheptulose-1-7-phosphate (S7P), sedoheptulose-1,7-bisphosphate (SBP), glyceraldehdye-3-phosphate (G3P), dihydroxy-acetone-phosphate (DHAP), erythrose-4-phosphate (E4P).

### Example 6. NRF2/KEAP1 deregulation sensitizes cancer cells to GLS inhibition.

The NRF2/KEAP1 pathway regulates redox balance in part through the transcriptional regulation of GCLC, the enzyme required to convert glutamine-derived glutamate and cysteine into glutathione (Fig. 6). Figure 6 shows that cells with loss-of-function mutations in KEAPlor gain-of-function mutations in NRF2 become addicted to high levels of glutathione for coping with oxidative stress during conditions of rapid proliferation (Tumor Cell, Fig. 6A). When GLS is inhibited in these cells (Treated Tumor Cell, Fig. 6B), intracellular pools of glutamate are drastically reduced, and thus, glutathione synthesis is inhibited. This response shifts the redox balance of tumor cells causing an increase in ROS accumulation and DNA damage leading to cell death.

As the major intracellular antioxidant, glutathione provides reducing power to limit the mutagenic and DNA damaging effects of intracellular ROS. Tumors that harbor somatic mutations that deregulate the NRF2/KEAP1 pathway evolve a dependence on glutathione and an addiction on glutamine. GLS inhibition reduces the conversion of glutamine to glutamate and thus inhibits the synthesis of glutathione. By altering redox balance, NRF2/KEAP1 mutant cells accumulate ROS which induces DNA damage and induces cell death. This model explains the exquisite sensitivity of NRF2/KEAP1 mutant NSCLC cell lines to GLS inhibition.

## Claims

1. Glutaminase inhibitor selected from:
N,N'-(5,5'-(2,2'-sulfonylbis(ethane-2,1-diyl))bis(1,3,4-thiadiazole-5,2-diyl))bis(2-(pyridin-2-yl)acetamide),
N-methyl-1-{4-[6-(2-{4-[3-(trifluoromethoxy)phenyl]pyridin-2-yl}acetamido)pyridazin-3-yl]butyl}-1H-1,2,3-triazole-4-carboxamide,
1-(2-fluoro-4-(5-(2-(pyridin-2-yl)acetamido)-1,3,4-thiadiazol-2-yl)butyl)-N-((4-(trifluoromethyl)pyridin-2-yl)methyl)-1H-1,2,3-triazole-4-carboxamide,
1-(2-fluoro-4-(6-(2-(4-(trifluoromethyl)pyridin-2-yl)acetamido)pyridazin-3-yl)butyl)-N-methyl-1H-1,2,3-triazole-4-carboxamide,
N-(pyridin-2-ylmethyl)-5-(3-(6-(2-(3-(trifluoromethoxy)phenyl)acetamido)pyridazin-3-yl)pyrrolidin-1-yl)-1,3,4-thiadiazole-2-carboxamide,
(R)-1-(2-fluoro-4-(6-(2-(4-(3-(trifluoromethoxy)phenyl)pyridin-2-yl)acetamido)pyridazin-3-yl)butyl)-N-methyl-1H-1,2,3 -triazole-4-carboxamide,
(R)-1-(2-fluoro-4-(6-(2-(4-(trifluoromethyl)pyridin-2-yl)acetamido)pyridazin-3-yl)butyl)-N-methyl-1H-1,2,3-triazole-4-carboxamide,
(R)-1-(2-fluoro-4-(6-(2-(6-methyl-4-(trifluoromethyl)pyridin-2-yl)acetamido)pyridazin-3-yl)butyl)-N-methyl-1H-1,2,3-triazole-4-carboxamide,
(R)-1-(4-(6-(2-(4-(cyclopropyldifluoromethyl)pyridin-2-yl)acetamido)pyridazin-3-yl)-2-fluorobutyl)-N-methyl-1H-1,2,3-triazole-4-carboxamide,
(R)-1-(4-(6-(2-(4-(3,3-difluorocyclobutoxy)pyridin-2-yl)acetamido)pyridazin-3-yl)-2-fluorobutyl)-N-methyl-1H-1,2,3-triazole-4-carboxamide,
(R)-1-(2-fluoro-4-(6-(2-(1-(3-(trifluoromethoxy)phenyl)-1H-imidazol-4-yl)acetamido)pyridazin-3-yl)butyl)-N-methyl-1H-1,2,3-triazole-4-carboxamide,
1-(4-(6-(2-(4-cyclobutoxypyridin-2-yl)acetamido)pyridazin-3-yl)butyl)-N-methyl-1H-1,2,3-triazole-4-carboxamide,
1-(4-(6-(2-(4-cyclobutoxypyridin-2-yl)acetamido)pyridazin-3-yl)-2-fluorobutyl)-N-methyl-1H-1,2,3-triazole-4-carboxamide,
1-(4-(6-(2-(4-(3,3-difluorocyclobutoxy)pyridin-2-yl)acetamido)pyridazin-3-yl)butyl)-N-methyl-1H-1,2,3-triazole-4-carboxamide,
1-(4-(6-(2-(4-(3,3-difluorocyclobutoxy)pyridin-2-yl)acetamido)pyridazin-3-yl)-2-fluorobutyl)-N-methyl-1H-1,2,3-triazole-4-carboxamide,
(R)-1-(4-(6-(2-(4-cyclopropylpyridin-2-yl)acetamido)pyridazin-3-yl)-2-fluorobutyl)-N-methyl-1H-1,2,3-triazole-4-carboxamide,
5-(3-(6-(2-(pyridin-2-yl)acetamido)pyridazin-3-yl)pyrrolidin-1-yl)-N-((4-(trifluoromethyl)pyridin-2-yl)methyl)-1,3,4-thiadiazole-2-carboxamide, and
N,N'-(5,5'-(cyclohexane-1,3-diyl)bis(1,3,4-thiadiazole-5,2-diyl))bis(2-phenylacetamide),
or a salt thereof, for use in the treatment of lung cancer wherein the NRF2/KEAP1 pathway in the cancer cells is deregulated.

2. Glutaminase inhibitor for use according to claim 1 wherein lung cancer is non-small cell lung cancer.

3. Glutaminase inhibitor for use according to claim 2 wherein the cancer has a mutation in either NRF2 or KEAP1.

4. Glutaminase inhibitor for use according to claim 3, wherein the mutation is chosen from KEAP1-G333C; KEAP1-D236H; KEAP1-A170_R204del; KEAP1-G364C; NFE2L2-G31A; KEAP1-G462W; KEAP1-R272L; KEAP1-V568F; KEAP1-W252C; KEAP1-R336 and NFELE2-D77N together with NFELE2-D77_E79delDEE.

5. Glutaminase inhibitor for use according to claim 3, wherein the mutation is a somatic mutation.

6. Glutaminase inhibitor for use according to claim 5, wherein the somatic mutation is in KEAP1 or the KEAP1 binding domain of NRF2.

7. Glutaminase inhibitor for use according to claim 3, wherein the cancer has a loss-of-function mutation in KEAP1, or a gain-of-function mutation in NRF2.

## Patentansprüche

1. Glutaminase-Hemmer, ausgewählt aus:
N,N'-(5,5'-(2,2'-Sulfonylbis(ethan-2,1-diyl))bis(1,3,4-thiadiazol-5,2-diyl))bis(2-(pyridin-2-yl)acetamid),
N-Methyl-1-{4-[6-(2-{4-[3-(trifluormethoxy)phenyl]pyridin-2-yl}acetamido)pyridazin-3-yl]butyl}-1H-1,2,3-triazol-4-carboxamid,
1-(2-Fluor-4-(5-(2-(pyridin-2-yl)acetamido)-1,3,4-thiadiazol-2-yl)butyl)-N-((4-(trifluormethyl)pyridin-2-yl)methyl)-1H-1,2,3-triazol-4-carboxamid,
1-(2-Fluor-4-(6-(2-(4-(trifluormethyl)pyridin-2-yl)acetamido)pyridazin-3-yl)butyl)-N-methyl-1H-1,2,3-triazol-4-carboxamid,
N-(Pyridin-2-ylmethyl)-5-(3-(6-(2-(3-(trifluormethoxy)phenyl)acetamido)pyridazin-3-yl)pyrrolidin-1-yl)-1,3,4-thiadiazol-2-carboxamid,
(R)-1-(2-Fluor-4-(6-(2-(4-(3-(trifluormethoxy)phenyl)pyridin-2-yl)acetamido)pyridazin-3-yl)butyl)-N-methyl-1H-1,2,3-triazol-4-carboxamid,
(R)-1-(2-Fluor-4-(6-(2-(4-(trifluormethyl)pyridin-2-yl)acetamido)pyridazin-3-yl)butyl)-N-methyl-1H-1,2,3-triazol-4-carboxamid,
(R)-1-(2-Fluor-4-(6-(2-(6-methyl-4-(trifluormethyl)pyridin-2-yl)acetamido)pyridazin-3-yl)butyl)-N-methyl-1H-1,2,3-triazol-4-carboxamid,
(R)-1-(4-(6-(2-(4-(Cyclopropyldifluormethyl)pyridin-2-yl)acetamido)pyridazin-3-yl)-2-fluorbutyl)-N-methyl-1H-1,2,3-triazol-4-carboxamid,
(R)-1-(4-(6-(2-(4-(3,3-Difluorcyclobutoxy)pyridin-2-yl)acetamido)pyridazin-3-yl)-2-fluorbutyl)-N-methyl-1H-1,2,3-triazol-4-carboxamid,
(R)-1-(2-Fluor-4-(6-(2-(1-(3-(trifluormethoxy)phenyl)-1H-imidazo-4-yl)acetamido)pyridazin-3-yl)butyl)-N-methyl-1H-1,2,3-triazol-4-carboxamid,
1-(4-(6-(2-(4-Cyclobutoxypyridin-2-yl)acetamido)pyridazin-3-yl)butyl)-N-methyl-1H-1,2,3-triazol-4-carboxamid,
1-(4-(6-(2-(4-Cyclobutoxypyridin-2-yl)acetamido)pyridazin-3-yl)-2-fluorbutyl)-N-methyl-1H-1,2,3-triazol-4-carboxamid,
1-(4-(6-(2-(4-(3,3-difluorcyclobutoxy)pyridin-2-yl)acetamido)pyridazin-3-yl)butyl)-N-methyl-1H-1,2,3-triazol-4-carboxamid,
1-(4-(6-(2-(4-(3,3-Difluorcyclobutoxy)pyridin-2-yl)acetamido)pyridazin-3-yl)-2-fluorbutyl)-N-methyl-1H-1,2,3-triazol-4-carboxamid,
(R)-1-(4-(6-(2-(4-Cyclopropylpyridin-2-yl)acetamido)pyridazin-3-yl)-2-fluorbutyl)-N-methyl-1H-1,2,3-triazol-4-carboxamid,
5-(3-(6-(2-(Pyridin-2-yl)acetamido)pyridazin-3-yl)pyrrolidin-1-yl)-N-((4-(trifluormethyl)pyridin-2-yl)methyl)-1,3,4-thiadiazol-2-carboxamid, und
N,N'-(5,5'-(Cyclohexan-1,3-diyl)bis(1,3,4-thiadiazol-5,2-diyl))bis(2-phenylacetamid),
oder ein Salz davon, zur Verwendung bei der Behandlung von Lungenkrebs, wobei die NRF2/KEAP1-Bahn in den Krebszellen dereguliert ist.

2. Glutaminase-Hemmer zur Verwendung nach Anspruch 1, wobei Lungenkrebs nichtkleinzelliger Lungenkrebs ist.

3. Glutaminase-Hemmer zur Verwendung nach Anspruch 2, wobei der Krebs eine Mutation entweder in NRF2 oder KEAP1 aufweist.

4. Glutaminase-Hemmer zur Verwendung nach Anspruch 3, wobei die Mutation ausgewählt ist aus KEAP1-G333C; KEAP1-D236H; KEAP1-A170_R204del; KEAP1-G364C; NFE2L2-G31A; KEAP1-G462W; KEAP1-R272L; KEAP1-V568F; KEAP1-W252C; KEAP1-R336 und NFELE2-D77N zusammen mit NFELE2-D77_E79delDEE.

5. Glutaminase-Hemmer zur Verwendung nach Anspruch 3, wobei die Mutation eine somatische Mutation ist.

6. Glutaminase-Hemmer zur Verwendung nach Anspruch 5, wobei die somatische Mutation in KEAP1 oder der KEAP1-Bindungsdomäne von NRF2 ist.

7. Glutaminase-Hemmer zur Verwendung nach Anspruch 3, wobei der Krebs eine Funktionsverlustmutation in KEAP1 oder eine Funktionsgewinnmutation in NRF2 aufweist.

## Revendications

1. Inhibiteur de la glutaminase, choisi parmi :
N,N'-(5,5'-(2,2'-sulfonylbis(éthane-2,1-diyl))bis(1,3,4-thiadiazole-5,2-diyl))bis(2-(pyridin-2-yl)acétamide),
N-méthyl-1-{4-[6-(2-{4-[3-(trifluorométhoxy)phényl]pyridin-2-yl}acétamido)pyridazin-3-yl]butyl}-1H-1,2,3-triazole-4-carboxamide,
1-(2-fluoro-4-(5-(2-(pyridin-2-yl)acétamido)-1,3,4-thiadiazol-2-yl)butyl)-N-((4-(trifluorométhyl)pyridin-2-yl)méthyl)-1H-1,2,3-triazole-4-carboxamide,
1-(2-fluoro-4-(6-(2-(4-(trifluorométhyl)pyridin-2-yl)acétamido)pyridazin-3-yl)butyl)-N-méthyl-1H-1,2,3-triazole-4-carboxamide,
N-(pyridin-2-ylméthyl)-5-(3-(6-(2-(3-(trifluorométhoxy)phényl)acétamido)pyridazin-3-yl)pyrrolidin-1-yl)-1,3,4-thiadiazole-2-carboxamide,
(R)-1-(2-fluoro-4-(6-(2-(4-(3-(trifluorométhoxy)phényl)pyridin-2-yl)acétamido)pyridazin-3-yl)butyl)-N-méthyl-1H-1,2,3-triazole-4-carboxamide,
(R)-1-(2-fluoro-4-(6-(2-(4-(trifluorométhyl)pyridin-2-yl)acétamido)pyridazin-3-yl)butyl)-N-méthyl-1H-1,2,3-triazole-4-carboxamide,
(R)-1-(2-fluoro-4-(6-(2-(6-méthyl-4-(trifluorométhyl)pyridin-2-yl)acétamido)pyridazin-3-yl)butyl)-N-méthyl-1H-1,2,3-triazole-4-carboxamide,
(R)-1-(4-(6-(2-(4-(cyclopropyldifluorométhyl)pyridin-2-yl)acétamido)pyridazin-3-yl)-2-fluorobutyl)-N-méthyl-1H-1 ,2,3-triazole-4-carboxamide,
(R)-1-(4-(6-(2-(4-(3,3-difluorocyclobutoxy)pyridin-2-yl)acétamido)pyridazin-3-yl)-2-fluorobutyl)-N-méthyl-1H-1,2,3-triazole-4-carboxamide,
(R)-1-(2-fluoro-4-(6-(2-(1-(3-(trifluorométhoxy)phényl)-1H-imidazol-4-yl)acétamido)pyridazin-3-yl)butyl)-N-méthyl-1H-1,2,3-triazole-4-carboxamide,
1-(4-(6-(2-(4-cyclobutoxypyridin-2-yl)acétamido)pyridazin-3-yl)butyl)-N-méthyl-1H-1,2,3-triazole-4-carboxamide,
1-(4-(6-(2-(4-cyclobutoxypyridin-2-yl)acétamido)pyridazin-3-yl)-2-fluorobutyl)-N-méthyl-1H-1,2,3-triazole-4-carboxamide,
1-(4-(6-(2-(4-(3,3-difluorocyclobutoxy)pyridin-2-yl)acétamido)pyridazin-3-yl)butyl)-N-méthyl-1H-1,2,3-triazole-4-carboxamide,
1-(4-(6-(2-(4-(3,3-difluorocyclobutoxy)pyridin-2-yl)acétamido)pyridazin-3-yl)-2-fluorobutyl)-N-méthyl-1H-1,2,3-triazole-4-carboxamide,
(R)-1-(4-(6-(2-(4-cyclopropylpyridin-2-yl)acétamido)pyridazin-3-yl)-2-fluorobutyl)-N-méthyl-1H-1,2,3-triazole-4-carboxamide,
5-(3-(6-(2-(pyridin-2-yl)acétamido)pyridazin-3-yl)pyrrolidin-1-yl)-N-((4-(trifluorométhyl)pyridin-2-yl)méthyl)-1,3,4-thiadiazole-2-carboxamide, et
N,N'-(5,5'-(cyclohexane-1,3-diyl)bis(1,3,4-thiadiazole-5,2-diyl))bis(2-phénylacétamide),
ou un sel correspondant, destiné à être utilisé dans le traitement d'un cancer du poumon, la voie de NRF2/KEAP1 dans les cellules cancéreuses étant dérégulée.

2. Inhibiteur de la glutaminase destiné à être utilisé selon la revendication 1, dans lequel le cancer du poumon est un cancer du poumon non à petites cellules.

3. Inhibiteur de la glutaminase destiné à être utilisé selon la revendication 2, dans lequel le cancer présente une mutation dans NRF2 ou KEAP1.

4. Inhibiteur de la glutaminase destiné à être utilisé selon la revendication 3, dans lequel la mutation est choisie parmi KEAP1-G333C ; KEAP1-D236H ; KEAPI-A170_R204del ; KEAP1-G364C ; NFE2L2-G31A ; KEAP1-G462W ; KEAP1-R272L ; KEAP1-V568F ; KEAP1-W252C ; KEAP1-R336 et NFELE2-D77N conjointement avec NFELE2-D77_E79delDEE.

5. Inhibiteur de la glutaminase destiné à être utilisé selon la revendication 3, dans lequel la mutation est une mutation somatique.

6. Inhibiteur de la glutaminase destiné à être utilisé selon la revendication 5, dans lequel la mutation somatique est dans KEAP1 ou dans le domaine de liaison à KEAP1 de NRF2.

7. Inhibiteur de la glutaminase destiné à être utilisé selon la revendication 3, dans lequel le cancer présente une mutation de perte de fonction dans KEAP1 ou une mutation de gain de fonction dans NRF2.
